# EUROPEAN PATENT APPLICATION

(11) **EP 4 227 303 A1**
(43) Date of publication of application: **16.08.2023**
(21) Application number: 21877821.5
(22) Date of filing: 09.08.2021
(51) Int. Cl.: C07D 409/14, C07D 495/04, C07D 491/048, H01L 51/00, H01L 51/50

(54) **HETEROCYCLIC COMPOUND, ORGANIC LIGHT-EMITTING DEVICE COMPRISING SAME, MANUFACTURING METHOD THEREFOR, AND COMPOSITION FOR ORGANIC LAYER OF ORGANIC LIGHT-EMITTING DEVICE**

(30) Priority: 08.10.2020 KR 20200130395
(71) Applicant: LT Materials Co., Ltd., Yongin-si, Gyeonggi-do 17118 (KR)
(72) Inventor: KWON, Su-Jin, Yongin-si, Gyeonggi-do 17118 (KR); KIM, Ji-Young, Yongin-si, Gyeonggi-do 17118 (KR); MO, Jun-Tae, Yongin-si, Gyeonggi-do 17118 (KR); KIM, Dong-Jun, Yongin-si, Gyeonggi-do 17118 (KR)
(74) Representative: Goddar, Heinz J.
(86) International application number: PCT/KR2021/010498
(87) International publication number: WO 2022/075566

(57) **Abstract**

The present specification relates to a heterocyclic compound represented by Chemical Formula 1, an organic light emitting device comprising the same, a method for manufacturing the same, and a composition for an organic material layer.

## Description

### [Technical Field]

This application claims priority to and the benefits of Korean Patent Application No. 10-2020-0130395, filed with the Korean Intellectual Property Office on October 8, 2020, the entire contents of which are incorporated herein by reference.

The present specification relates to a heterocyclic compound, an organic light emitting device comprising the same, a method for manufacturing the same, and a composition for an organic material layer.

### [Background Art]

An organic electroluminescent device is one type of self-emissive display devices, and has an advantage of having a wide viewing angle, and a high response speed as well as having an excellent contrast.

An organic light emitting device has a structure disposing an organic thin film between two electrodes. When a voltage is applied to an organic light emitting device having such a structure, electrons and holes injected from the two electrodes bind and pair in the organic thin film, and light emits as these annihilate. The organic thin film may be formed in a single layer or a multilayer as necessary.

A material of the organic thin film may have a light emitting function as necessary. For example, as a material of the organic thin film, compounds capable of forming a light emitting layer themselves alone may be used, or compounds capable of performing a role of a host or a dopant of a host-dopant-based light emitting layer may also be used. In addition thereto, compounds capable of performing roles of hole injection, hole transfer, electron blocking, hole blocking, electron transfer, electron injection and the like may also be used as a material of the organic thin film.

Development of an organic thin film material has been continuously required for enhancing performance, lifetime or efficiency of an organic light emitting device.

### Prior Art Documents

### Patent Documents

US Patent No. 4,356,429

### [Disclosure]

### [Technical Problem]

The present disclosure is directed to providing a heterocyclic compound, an organic light emitting device comprising the same, a method for manufacturing the same, and a composition for an organic material layer.

### [Technical Solution]

One embodiment of the present application provides a heterocyclic compound represented by the following Chemical Formula 1. 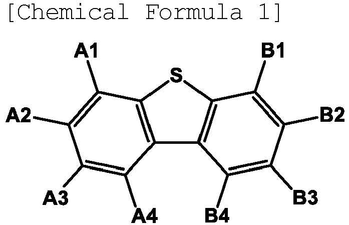 In Chemical Formula 1,
at least one of A1 to A4 is represented by the following Chemical Formula 1-1,
at least one of B1 to B4 is represented by the following Chemical Formula 1-2, and
substituents other than Chemical Formula 1-1 among A1 to A4 and substituents other than Chemical Formula 1-2 among B1 to B4 are the same as or different from each other, and each independently selected from the group consisting of hydrogen; deuterium; a halogen group; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -SiRR'R"; -P (=O) RR' ; and -NRR', or two or more groups adjacent to each other bond to each other to form a substituted or unsubstituted C6 to C60 aromatic hydrocarbon ring or a substituted or unsubstituted C2 to C60 heteroring, 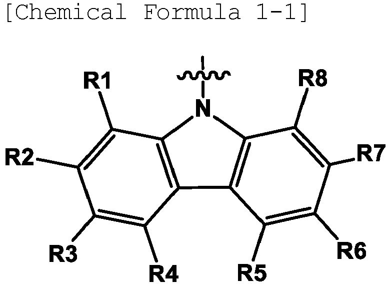 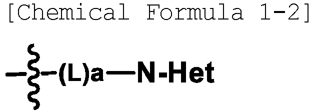
in Chemical Formula 1-1 and Chemical Formula 1-2, 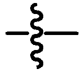 means a substituted position,
R1 to R8 are the same as or different from each other, and each independently selected from the group consisting of hydrogen; deuterium; a halogen group; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; - SiRR'R"; -P(=O)RR'; and -NRR', or two or more groups adjacent to each other bond to each other to form a substituted or unsubstituted C6 to C60 aromatic hydrocarbon ring or a substituted or unsubstituted C2 to C60 heteroring,
N-Het is a monocyclic or polycyclic C2 to C60 heterocyclic group substituted or unsubstituted and comprising one or more Ns,
L is a direct bond; a substituted or unsubstituted C6 to C60 arylene group; or a substituted or unsubstituted C2 to C60 heteroarylene group,
R, R' and R" are the same as or different from each other, and each independently hydrogen; a substituted or unsubstituted C1 to C60 alkyl group; or a substituted or unsubstituted C6 to C60 aryl group,
a is an integer of 0 to 3, and
when A3 is substituted with Chemical Formula 1-1 in Chemical Formula 1, any one of B1, B2 and B4 of Chemical Formula 1 is substituted with Chemical Formula 1-2.

In addition, one embodiment of the present application provides an organic light emitting device comprising a first electrode; a second electrode provided opposite to the first electrode; and one or more organic material layers provided between the first electrode and the second electrode, wherein one or more layers of the organic material layers comprise the heterocyclic compound represented by Chemical Formula 1.

In addition, in the organic light emitting device provided in one embodiment of the present application, the organic material layer comprising the heterocyclic compound of Chemical Formula 1 further comprises a heterocyclic compound represented by the following Chemical Formula 2. 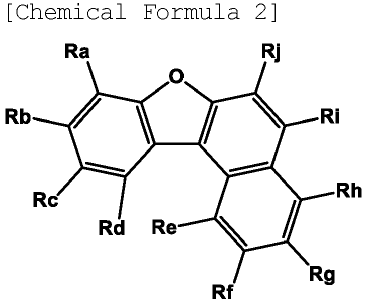 In Chemical Formula 2,
Ra to Rj are the same as or different from each other, and each independently selected from the group consisting of hydrogen; deuterium; a halogen group; -CN; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -SiR101R102R103; - P(=O)R101R102; and -NR101R102, or two or more groups adjacent to each other bond to each other to form a substituted or unsubstituted C6 to C60 aromatic hydrocarbon ring or a substituted or unsubstituted C2 to C60 heteroring,
R101, R102 and R103 are the same as or different from each other, and each independently hydrogen; deuterium; -CN; a substituted or unsubstituted C1 to C60 alkyl group; a substituted or unsubstituted C6 to C60 aryl group; or a substituted or unsubstituted C2 to C60 heteroaryl group, and
at least one of Ra to Rj is a substituted or unsubstituted C2 to C60 heteroaryl group.

In addition, another embodiment of the present application provides a composition for an organic material layer of an organic light emitting device, the composition comprising the heterocyclic compound represented by Chemical Formula 1 and the heterocyclic compound represented by Chemical Formula 2.

Lastly, one embodiment of the present application provides a method for manufacturing an organic light emitting device, the method comprising preparing a substrate; forming a first electrode on the substrate; forming one or more organic material layers on the first electrode; and forming a second electrode on the organic material layer, wherein the forming of organic material layers comprises forming one or more organic material layers using the composition for an organic material layer according to one embodiment of the present application.

### [Advantageous Effects]

A compound described in the present specification can be used as a material of an organic material layer of an organic light emitting device. In the organic light emitting device, the compound is capable of performing a role of a hole injection material, a hole transfer material, a light emitting material, an electron transfer material, an electron injection material or the like. Particularly, the compound can be used as a light emitting layer material of an organic light emitting device.

Specifically, the compound can be used alone as a light emitting material, or as a host material or a dopant material of a light emitting layer. Using the compound represented by Chemical Formula 1 in an organic material layer can lower a driving voltage, enhance light efficiency, and enhance lifetime properties of a device by thermal stability of the compound.

In addition, in the compound of Chemical Formula 1 according to the disclosure of the present application, one side benzene ring of the dibenzothiophene is represented by Chemical Formula 1-1 and the other side benzene ring is represented by Chemical Formula 1-2, and properties of excellent thermal stability is obtained by expanding a conjugation range around the core structure, and superior properties as a p-type host material are obtained when used in a device due to strong hole properties.

### [Description of Drawings]

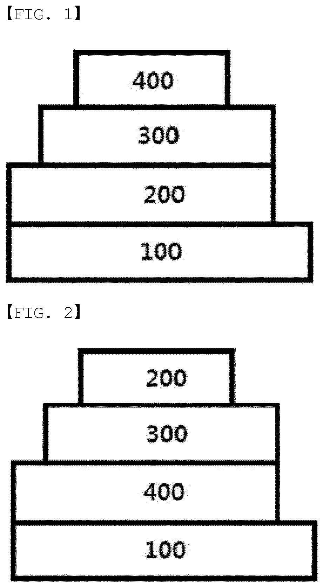

FIG. 1 to FIG. 3 are diagrams each schematically illustrating a lamination structure of an organic light emitting device according to one embodiment of the present application.

### [Reference Numeral]

100: Substrate
200: Anode
300: Organic Material Layer
301: Hole Injection Layer
302: Hole Transfer Layer
303: Light Emitting Layer
304: Hole Blocking Layer
305: Electron Transfer Layer
306: Electron Injection Layer
400: Cathode

### [Mode for Disclosure]

Hereinafter, the present application will be described in detail.

In the present specification, a term "substitution" means a hydrogen atom bonding to a carbon atom of a compound being changed to another substituent, and the position of substitution is not limited as long as it is a position at which the hydrogen atom is substituted, that is, a position at which a substituent can substitute, and when two or more substituents substitute, the two or more substituents may be the same as or different from each other.

In the present specification, "substituted or unsubstituted" means being substituted with one or more substituents selected from the group consisting of C1 to C60 linear or branched alkyl; C2 to C60 linear or branched alkenyl; C2 to C60 linear or branched alkynyl; C3 to C60 monocyclic or polycyclic cycloalkyl; C2 to C60 monocyclic or polycyclic heterocycloalkyl; C6 to C60 monocyclic or polycyclic aryl; C2 to C60 monocyclic or polycyclic heteroaryl; -SiRR'R"; - P(=O)RR'; C1 to C20 alkylamine; C6 to C60 monocyclic or polycyclic arylamine; and C2 to C60 monocyclic or polycyclic heteroarylamine, or being unsubstituted, or being substituted with a substituent linking two or more substituents selected from among the substituents illustrated above, or being unsubstituted.

In one embodiment of the present application, R, R' and R" are the same as or different from each other, and may be each independently a substituted or unsubstituted C1 to C60 alkyl group; a substituted or unsubstituted C6 to C60 aryl group; or a substituted or unsubstituted C2 to C60 heteroaryl group.

In another embodiment, R, R' and R" are the same as or different from each other, and may be each independently a substituted or unsubstituted C1 to C60 alkyl group; or a substituted or unsubstituted C6 to C60 aryl group.

In another embodiment, R, R' and R" are the same as or different from each other, and may be each independently a C1 to C60 alkyl group; or a C6 to C60 aryl group.

In another embodiment, R, R' and R" are the same as or different from each other, and may be each independently a methyl group; or a phenyl group.

In another embodiment, R, R' and R" may be a phenyl group.

In the present specification, "the number of protons" means the number of substituents that a specific compound may have, and specifically, the number of protons may mean the number of hydrogens. For example, the number of protons in unsubstituted benzene may be expressed as 5, the number of protons in an unsubstituted naphthyl group may be expressed as 7, the number of protons in a naphthyl group substituted with a phenyl group may be expressed as 6, and the number of protons in an unsubstituted biphenyl group may be expressed as 9.

In the present specification, a "case of a substituent being not indicated in a chemical formula or compound structure" means that a hydrogen atom bonds to a carbon atom. However, since deuterium (²H) is an isotope of hydrogen, some hydrogen atoms may be deuterium.

In one embodiment of the present application, a "case of a substituent being not indicated in a chemical formula or compound structure" may mean that positions that may come as a substituent may all be hydrogen or deuterium. In other words, since deuterium is an isotope of hydrogen, some hydrogen atoms may be deuterium that is an isotope, and herein, a content of the deuterium may be from 0% to 100%.

In one embodiment of the present application, in a "case of a substituent being not indicated in a chemical formula or compound structure", hydrogen and deuterium may be mixed in compounds when deuterium is not explicitly excluded such as a deuterium content being 0% or a hydrogen content being 100%. In other words, an expression of "substituent X is hydrogen" does not exclude deuterium unlike a hydrogen content being 100% or a deuterium content being 0%, and therefore, may mean a state in which hydrogen and deuterium are mixed.

In one embodiment of the present application, deuterium is one of isotopes of hydrogen, is an element having deuteron formed with one proton and one neutron as a nucleus, and may be expressed as hydrogen-2, and the elemental symbol may also be written as D or ²H.

In one embodiment of the present application, an isotope means an atom with the same atomic number (Z) but with a different mass number (A), and may also be interpreted as an element with the same number of protons but with a different number of neutrons.

In one embodiment of the present application, a meaning of a content T% of a specific substituent may be defined as T2/T1×100=T% when the total number of substituents that a basic compound may have is defined as T1, and the number of specific substituents among these is defined as T2.

In other words, in one example, having a deuterium content of 20% in a phenyl group represented by 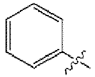 means that the total number of substituents that the phenyl group may have is 5 (T1 in the formula), and the number of deuterium among these is 1 (T2 in the formula). In other words, having a deuterium content of 20% in a phenyl group may be represented by the following structural formulae.

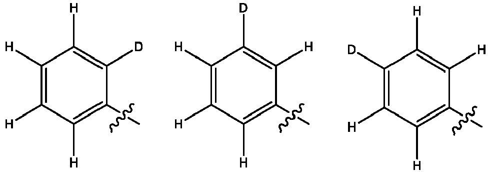

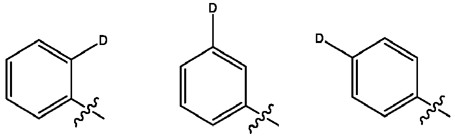

In addition, in one embodiment of the present application, "a phenyl group having a deuterium content of 0%" may mean a phenyl group that does not comprise a deuterium atom, that is, a phenyl group that has 5 hydrogen atoms.

In the present specification, the halogen may be fluorine, chlorine, bromine or iodine.

In the present specification, the alkyl group comprises linear or branched having 1 to 60 carbon atoms, and may be further substituted with other substituents. The number of carbon atoms of the alkyl group may be from 1 to 60, specifically from 1 to 40 and more specifically from 1 to 20. Specific examples thereof may comprise a methyl group, an ethyl group, a propyl group, an n-propyl group, an isopropyl group, a butyl group, an n-butyl group, an isobutyl group, a tert-butyl group, a sec-butyl group, a 1-methyl-butyl group, a 1-ethylbutyl group, a pentyl group, an n-pentyl group, an isopentyl group, a neopentyl group, a tert-pentyl group, a hexyl group, an n-hexyl group, a 1-methylpentyl group, a 2-methylpentyl group, a 4-methyl-2-pentyl group, a 3,3-dimethylbutyl group, a 2-ethylbutyl group, a heptyl group, an n-heptyl group, a 1-methylhexyl group, a cyclopentylmethyl group, a cyclohexylmethyl group, an octyl group, an n-octyl group, a tert-octyl group, a 1-methylheptyl group, a 2-ethylhexyl group, a 2-propylpentyl group, an n-nonyl group, a 2,2-dimethylheptyl group, a 1-ethyl-propyl group, a 1,1-dimethyl-propyl group, an isohexyl group, a 2-methylpentyl group, a 4-methylhexyl group, a 5-methylhexyl group and the like, but are not limited thereto.

In the present specification, the alkenyl group comprises linear or branched having 2 to 60 carbon atoms, and may be further substituted with other substituents. The number of carbon atoms of the alkenyl group may be from 2 to 60, specifically from 2 to 40 and more specifically from 2 to 20. Specific examples thereof may comprise a vinyl group, a 1-propenyl group, an isopropenyl group, a 1-butenyl group, a 2-butenyl group, a 3-butenyl group, a 1-pentenyl group, a 2-pentenyl group, a 3-pentenyl group, a 3-methyl-1-butenyl group, a 1,3-butadienyl group, an allyl group, a 1-phenylvinyl-1-yl group, a 2-phenylvinyl-1-yl group, a 2,2-diphenylvinyl-1-yl group, a 2-phenyl-2-(naphthyl-1-yl)vinyl-1-yl group, a 2,2-bis(diphenyl-1-yl)vinyl-1-yl group, a stilbenyl group, a styrenyl group and the like, but are not limited thereto.

In the present specification, the alkynyl group comprises linear or branched having 2 to 60 carbon atoms, and may be further substituted with other substituents. The number of carbon atoms of the alkynyl group may be from 2 to 60, specifically from 2 to 40 and more specifically from 2 to 20.

In the present specification, the alkoxy group may be linear, branched or cyclic. The number of carbon atoms of the alkoxy group is not particularly limited, but is preferably from 1 to 20. Specific examples thereof may comprise methoxy, ethoxy, n-propoxy, isopropoxy, n-butoxy, isobutoxy, tert-butoxy, sec-butoxy, n-pentyloxy, neopentyloxy, isopentyloxy, n-hexyloxy, 3,3-dimethylbutyloxy, 2-ethylbutyloxy, n-octyloxy, n-nonyloxy, n-decyloxy, benzyloxy, p-methylbenzyloxy and the like, but are not limited thereto.

In the present specification, the cycloalkyl group comprises a monocyclic or polycyclic aryl group having 3 to 60 carbon atoms, and may be further substituted with other substituents. Herein, the polycyclic means a group in which the cycloalkyl group is directly linked to or fused with other cyclic groups. Herein, the other cyclic groups may be a cycloalkyl group, but may also be different types of cyclic groups such as a heterocycloalkyl group, an aryl group and a heteroaryl group. The number of carbon groups of the cycloalkyl group may be from 3 to 60, specifically from 3 to 40 and more specifically from 5 to 20. Specific examples thereof may comprise a cyclopropyl group, a cyclobutyl group, a cyclopentyl group, a 3-methylcyclopentyl group, a 2,3-dimethylcyclopentyl group, a cyclohexyl group, a 3-methylcyclohexyl group, a 4-methylcyclohexyl group, a 2,3-dimethylcyclohexyl group, a 3,4,5-trimethylcyclohexyl group, a 4-tert-butylcyclohexyl group, a cycloheptyl group, a cyclooctyl group and the like, but are not limited thereto.

In the present specification, the heterocycloalkyl group comprises O, S, Se, N or Si as a heteroatom, comprises monocyclic or polycyclic having 2 to 60 carbon atoms, and may be further substituted with other substituents. Herein, the polycyclic means a group in which the heterocycloalkyl group is directly linked to or fused with other cyclic groups. Herein, the other cyclic groups may be a heterocycloalkyl group, but may also be different types of cyclic groups such as a cycloalkyl group, an aryl group and a heteroaryl group. The number of carbon atoms of the heterocycloalkyl group may be from 2 to 60, specifically from 2 to 40 and more specifically from 3 to 20.

In the present specification, the aryl group comprises monocyclic or polycyclic having 6 to 60 carbon atoms, and may be further substituted with other substituents. Herein, the polycyclic means a group in which the aryl group is directly linked to or fused with other cyclic groups. Herein, the other cyclic groups may be an aryl group, but may also be different types of cyclic groups such as a cycloalkyl group, a heterocycloalkyl group and a heteroaryl group. The aryl group comprises a spiro group. The number of carbon atoms of the aryl group may be from 6 to 60, specifically from 6 to 40 and more specifically from 6 to 25. Specific examples of the aryl group may comprise a phenyl group, a biphenyl group, a triphenyl group, a naphthyl group, an anthryl group, a chrysenyl group, a phenanthrenyl group, a perylenyl group, a fluoranthenyl group, a triphenylenyl group, a phenalenyl group, a pyrenyl group, a tetracenyl group, a pentacenyl group, a fluorenyl group, an indenyl group, an acenaphthylenyl group, a benzofluorenyl group, a spirobifluorenyl group, a 2,3-dihydro-1H-indenyl group, a fused ring group thereof, and the like, but are not limited thereto.

In the present specification, the phosphine oxide group is represented by -P(=O)R201R202, R201 and R202 are the same as or different from each other and may be each independently a substituent formed with at least one of hydrogen; deuterium; a halogen group; an alkyl group; an alkenyl group; an alkoxy group; a cycloalkyl group; an aryl group; and a heterocyclic group. Specifically, the phosphine oxide group may be substituted with an aryl group, and as the aryl group, the examples described above may be used. Examples of the phosphine oxide group may comprise a diphenylphosphine oxide group, a dinaphthylphosphine oxide group and the like, but are not limited thereto.

In the present specification, the silyl group is a substituent comprising Si, having the Si atom directly linked as a radical, and is represented by -SiR204R205R206. R204 to R206 are the same as or different from each other, and may be each independently a substituent formed with at least one of hydrogen; deuterium; a halogen group; an alkyl group; an alkenyl group; an alkoxy group; a cycloalkyl group; an aryl group; and a heterocyclic group. Specific examples of the silyl group may comprise a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group and the like, but are not limited thereto.

In the present specification, the fluorenyl group may be substituted, and adjacent substituents may bond to each other to form a ring.

When the fluorenyl group is substituted, 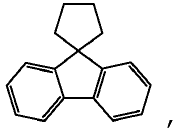 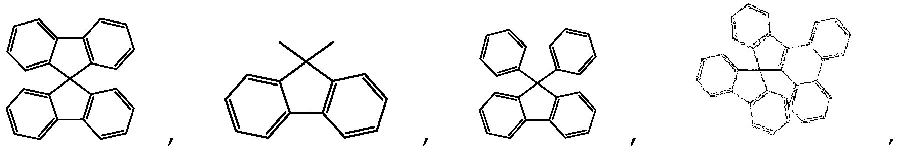 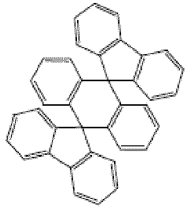 and the like may be included, however, the structure is not limited thereto.

In the present specification, the heteroaryl group comprises S, O, Se, N or Si as a heteroatom, comprises monocyclic or polycyclic having 2 to 60 carbon atoms, and may be further substituted with other substituents. Herein, the polycyclic means a group in which the heteroaryl group is directly linked to or fused with other cyclic groups. Herein, the other cyclic groups may be a heteroaryl group, but may also be different types of cyclic groups such as a cycloalkyl group, a heterocycloalkyl group and an aryl group. The number of carbon atoms of the heteroaryl group may be from 2 to 60, specifically from 2 to 40 and more specifically from 3 to 25. Specific examples of the heteroaryl group may comprise a pyridyl group, a pyrrolyl group, a pyrimidyl group, a pyridazinyl group, a furanyl group, a thiophene group, an imidazolyl group, a pyrazolyl group, an oxazolyl group, an isoxazolyl group, a thiazolyl group, an isothiazolyl group, a triazolyl group, a furazanyl group, an oxadiazolyl group, a thiadiazolyl group, a dithiazolyl group, a tetrazolyl group, a pyranyl group, a thiopyranyl group, a diazinyl group, an oxazinyl group, a thiazinyl group, a dioxynyl group, a triazinyl group, a tetrazinyl group, a quinolyl group, an isoquinolyl group, a quinazolinyl group, an isoquinazolinyl group, a qninozolinyl group, a naphthyridyl group, an acridinyl group, a phenanthridinyl group, an imidazopyridinyl group, a diazanaphthalenyl group, a triazaindene group, an indolyl group, an indolizinyl group, a benzothiazolyl group, a benzoxazolyl group, a benzimidazolyl group, a benzothiophene group, a benzofuran group, a dibenzothiophene group, a dibenzofuran group, a carbazolyl group, a benzocarbazolyl group, a dibenzocarbazolyl group, a phenazinyl group, a dibenzosilole group, spirobi(dibenzosilole), a dihydrophenazinyl group, a phenoxazinyl group, a phenanthridyl group, an imidazopyridinyl group, a thienyl group, an indolo[2,3-a]carbazolyl group, an indolo[2,3-b]carbazolyl group, an indolinyl group, a 10,11-dihydro-dibenzo[b,f]azepine group, a 9,10-dihydroacridinyl group, a phenanthrazinyl group, a phenothiathiazinyl group, a phthalazinyl group, a naphthylidinyl group, a phenanthrolinyl group, a benzo[c][1,2,5]thiadiazolyl group, a 5,10-dihydrobenzo[b,e][1,4]azasilinyl group, a pyrazolo[1,5-c]quinazolinyl group, a pyrido[1,2-b]indazolyl group, a pyrido[1,2-a]imidazo[1,2-e]indolinyl group, a 5,11-dihydroindeno[1,2-b]carbazolyl group and the like, but are not limited thereto.

In the present specification, the amine group may be selected from the group consisting of a monoalkylamine group; a monoarylamine group; a monoheteroarylamine group; -NH₂; a dialkylamine group; a diarylamine group; a diheteroarylamine group; an alkylarylamine group; an alkylheteroarylamine group; and an arylheteroarylamine group, and although not particularly limited thereto, the number of carbon atoms is preferably from 1 to 30. Specific examples of the amine group may comprise a methylamine group, a dimethylamine group, an ethylamine group, a diethylamine group, a phenylamine group, a naphthylamine group, a biphenylamine group, a dibiphenylamine group, an anthracenylamine group, a 9-methyl-anthracenylamine group, a diphenylamine group, a phenylnaphthylamine group, a ditolylamine group, a phenyltolylamine group, a triphenylamine group, a biphenylnaphthylamine group, a phenylbiphenylamine group, a biphenylfluorenylamine group, a phenyltriphenylenylamine group, a biphenyltriphenylenylamine group and the like, but are not limited thereto.

In the present specification, the arylene group means the aryl group having two bonding sites, that is, a divalent group. The descriptions on the aryl group provided above may be applied thereto except for those that are each a divalent group. In addition, the heteroarylene group means the heteroaryl group having two bonding sites, that is, a divalent group. The descriptions on the heteroaryl group provided above may be applied thereto except for those that are each a divalent group.

In the present specification, the "adjacent" group may mean a substituent substituting an atom directly linked to an atom substituted by the corresponding substituent, a substituent sterically most closely positioned to the corresponding substituent, or another substituent substituting an atom substituted by the corresponding substituent. For example, two substituents substituting ortho positions in a benzene ring, and two substituents substituting the same carbon in an aliphatic ring may be interpreted as groups "adjacent" to each other.

As the aliphatic or aromatic hydrocarbon ring or heteroring that adjacent groups may form, the structures illustrated as the cycloalkyl group, the cycloheteroalkyl group, the aryl group and the heteroaryl group described above may be used except for those that are not a monovalent group.

One embodiment of the present application provides a heterocyclic compound represented by Chemical Formula 1.

In one embodiment of the present application, the substituents other than Chemical Formula 1-1 among A1 to A4 and the substituents other than Chemical Formula 1-2 among B1 to B4 may be hydrogen.

In one embodiment of the present application, A1 of Chemical Formula 1 is represented by Chemical Formula 1-1, and A2 to A4 may be hydrogen.

In one embodiment of the present application, A2 of Chemical Formula 1 is represented by Chemical Formula 1-1, and A1, A3 and A4 may be hydrogen.

In one embodiment of the present application, A3 of Chemical Formula 1 is represented by Chemical Formula 1-1, and A1, A2 and A4 may be hydrogen.

In one embodiment of the present application, A4 of Chemical Formula 1 is represented by Chemical Formula 1-1, and A1 to A3 may be hydrogen.

In one embodiment of the present application, B1 of Chemical Formula 1 is represented by Chemical Formula 1-2, and B2 to B4 may be hydrogen.

In one embodiment of the present application, B2 of Chemical Formula 1 is represented by Chemical Formula 1-2, and B1, B3 and B4 may be hydrogen.

In one embodiment of the present application, B3 of Chemical Formula 1 is represented by Chemical Formula 1-2, and B1, B2 and B4 may be hydrogen.

In one embodiment of the present application, B4 of Chemical Formula 1 is represented by Chemical Formula 1-2, and B1 to B3 may be hydrogen.

In one embodiment of the present application, when B1 of Chemical Formula 1 is represented by Chemical Formula 1-2, one of A1 and A2 of Chemical Formula 1 may be represented by Chemical Formula 1-1.

In one embodiment of the present application, when B2 of Chemical Formula 1 is represented by Chemical Formula 1-2, one of A1 to A4 of Chemical Formula 1 may be represented by Chemical Formula 1-1.

In one embodiment of the present application, when B4 of Chemical Formula 1 is represented by Chemical Formula 1-2, A2 of Chemical Formula 1 may be represented by Chemical Formula 1-1.

In one embodiment of the present application, A1 of Chemical Formula 1 is represented by Chemical Formula 1-1, B1 of Chemical Formula 1 is represented by Chemical Formula 1-2, and A2 to A4 and B2 to B4 may be hydrogen.

In one embodiment of the present application, A1 of Chemical Formula 1 is represented by Chemical Formula 1-1, B2 of Chemical Formula 1 is represented by Chemical Formula 1-2, and A2 to A4 and B1, B3 and B4 may be hydrogen.

In one embodiment of the present application, A2 of Chemical Formula 1 is represented by Chemical Formula 1-1, B1 of Chemical Formula 1 is represented by Chemical Formula 1-2, and A1, A3, A4 and B2 to B4 may be hydrogen.

In one embodiment of the present application, A2 of Chemical Formula 1 is represented by Chemical Formula 1-1, B2 of Chemical Formula 1 is represented by Chemical Formula 1-2, and A1, A3, A4, B1, B3 and B4 may be hydrogen.

In one embodiment of the present application, A2 of Chemical Formula 1 is represented by Chemical Formula 1-1, B3 of Chemical Formula 1 is represented by Chemical Formula 1-2, and A1, A3, A4, B1, B2 and B4 may be hydrogen.

In one embodiment of the present application, A2 of Chemical Formula 1 is represented by Chemical Formula 1-1, B4 of Chemical Formula 1 is represented by Chemical Formula 1-2, and A1, A3, A4 and B1 to B3 may be hydrogen.

In one embodiment of the present application, A3 of Chemical Formula 1 is represented by Chemical Formula 1-1, B2 of Chemical Formula 1 is represented by Chemical Formula 1-2, and A1, A2, A4, B1, B3 and B4 may be hydrogen.

In one embodiment of the present application, A4 of Chemical Formula 1 is represented by Chemical Formula 1-1, B2 of Chemical Formula 1 is represented by Chemical Formula 1-2, and A1 to A3, B1, B3 and B4 may be hydrogen.

In one embodiment of the present application, R1 to R8 are the same as or different from each other, and each independently selected from the group consisting of hydrogen; deuterium; a halogen group; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -SiRR'R"; -P (=O) RR' ; and -NRR', or two or more groups adjacent to each other may bond to each other to form a substituted or unsubstituted C6 to C60 aromatic hydrocarbon ring or a substituted or unsubstituted C2 to C60 heteroring.

In another embodiment, R1 to R8 are the same as or different from each other, and each independently selected from the group consisting of hydrogen; deuterium; a substituted or unsubstituted C1 to C60 alkyl group; a substituted or unsubstituted C6 to C60 aryl group; and a substituted or unsubstituted C2 to C60 heteroaryl group, or two or more groups adjacent to each other may bond to each other to form a substituted or unsubstituted C6 to C60 aromatic hydrocarbon ring or a substituted or unsubstituted C2 to C60 heteroring.

In another embodiment, R1 to R8 are the same as or different from each other, and each independently selected from the group consisting of hydrogen; deuterium; a substituted or unsubstituted C1 to C60 alkyl group; a substituted or unsubstituted C6 to C60 aryl group; and a substituted or unsubstituted C2 to C60 heteroaryl group, or two or more groups adjacent to each other may bond to each other to form a substituted or unsubstituted C6 to C60 aromatic hydrocarbon ring.

In another embodiment, R1 to R8 are the same as or different from each other, and each independently selected from the group consisting of hydrogen; deuterium; a substituted or unsubstituted C1 to C40 alkyl group; a C6 to C40 aryl group unsubstituted or substituted with deuterium; and a substituted or unsubstituted C2 to C40 heteroaryl group, or two or more groups adjacent to each other may bond to each other to form a substituted or unsubstituted C6 to C40 aromatic hydrocarbon ring.

In another embodiment, R1 to R8 are the same as or different from each other, and each independently selected from the group consisting of hydrogen; deuterium; and a C6 to C40 aryl group unsubstituted or substituted with deuterium, or two or more groups adjacent to each other may bond to each other to form a monocyclic C6 to C40 aromatic hydrocarbon ring unsubstituted or substituted with deuterium.

In another embodiment, R1 to R8 are the same as or different from each other, and each independently selected from the group consisting of hydrogen; deuterium; and a monocyclic C6 to C40 aryl group unsubstituted or substituted with deuterium, or two or more groups adjacent to each other may bond to each other to form a monocyclic C6 to C40 aromatic hydrocarbon ring unsubstituted or substituted with deuterium.

In another embodiment, R1 to R8 are the same as or different from each other, and each independently hydrogen; deuterium; or a phenyl group unsubstituted or substituted with deuterium, or two or more groups adjacent to each other may bond to each other to form a benzene ring unsubstituted or substituted with deuterium.

In one embodiment of the present application, at least one of R1 to R4 is a substituted or unsubstituted C6 to C60 aryl group, or two or more groups adjacent to each other may bond to each other to form a substituted or unsubstituted C6 to C60 aromatic hydrocarbon ring.

In one embodiment of the present application, Chemical Formula 1-1 may be represented by any one of the following Chemical Formulae 1-1-1 to 1-1-8.

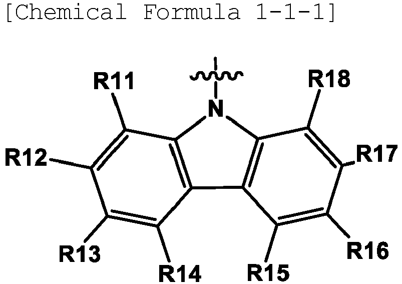

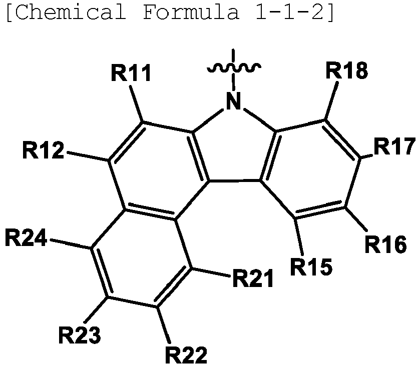

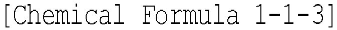

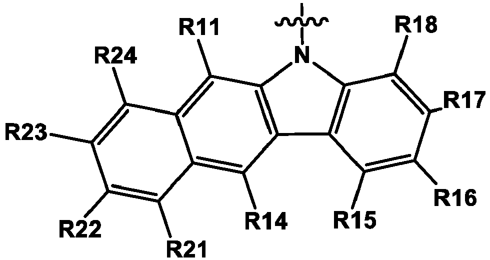

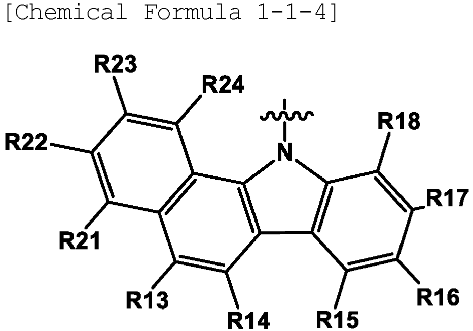

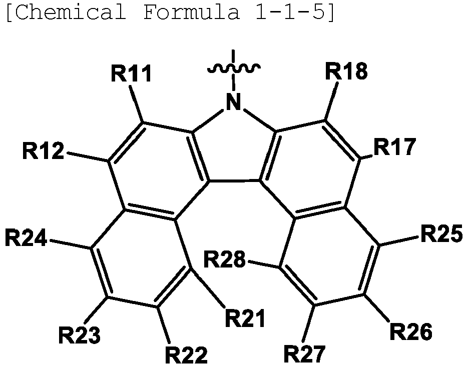

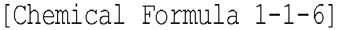

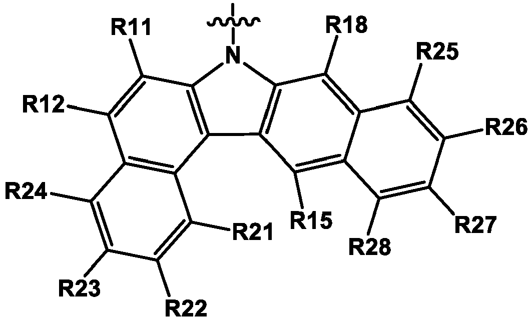

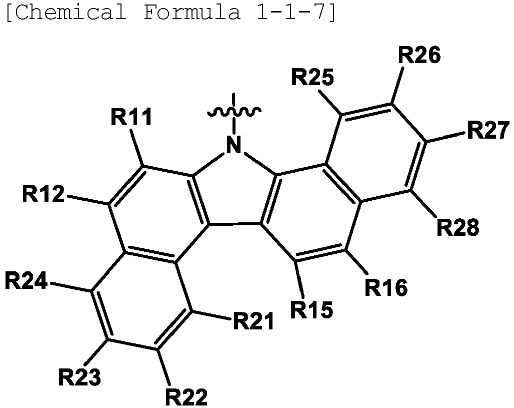

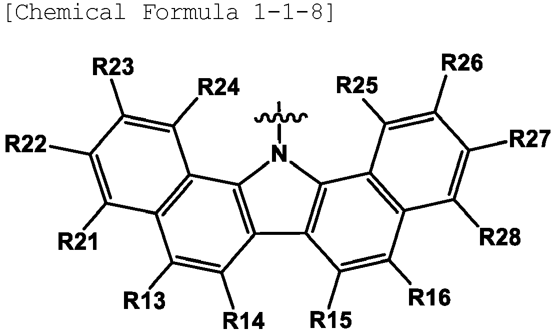

In Chemical Formulae 1-1-1 to 1-1-8,
R11 to R18 are the same as or different from each other, and each independently hydrogen; deuterium; a substituted or unsubstituted C1 to C60 alkyl group; a substituted or unsubstituted C6 to C60 aryl group; or a substituted or unsubstituted C2 to C60 heteroaryl group,
R21 to R28 are the same as or different from each other, and each independently selected from the group consisting of hydrogen; deuterium; a halogen group; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; - SiRR'R"; -P(=O)RR'; and -NRR', or two or more groups adjacent to each other bond to each other to form a substituted or unsubstituted C6 to C60 aromatic hydrocarbon ring or a substituted or unsubstituted C2 to C60 heteroring, and
R, R' and R" have the same definitions as in Chemical Formula 1.

In one embodiment of the present application, R11 to R18 are the same as or different from each other, and may be each independently hydrogen; deuterium; a substituted or unsubstituted C1 to C60 alkyl group; a substituted or unsubstituted C6 to C60 aryl group; or a substituted or unsubstituted C2 to C60 heteroaryl group.

In another embodiment, R11 to R18 are the same as or different from each other, and may be each independently hydrogen; deuterium; or a substituted or unsubstituted C6 to C60 aryl group.

In another embodiment, R11 to R18 are the same as or different from each other, and may be each independently hydrogen; deuterium; or a substituted or unsubstituted C6 to C40 aryl group.

In another embodiment, R11 to R18 are the same as or different from each other, and may be each independently hydrogen; deuterium; or a C6 to C30 aryl group unsubstituted or substituted with deuterium.

In another embodiment, R11 to R18 are the same as or different from each other, and may be each independently hydrogen; deuterium; or a monocyclic or polycyclic C6 to C30 aryl group unsubstituted or substituted with deuterium.

In another embodiment, R11 to R18 are the same as or different from each other, and may be each independently hydrogen; deuterium; a monocyclic C6 to C10 aryl group unsubstituted or substituted with deuterium; or a polycyclic C10 to C30 aryl group unsubstituted or substituted with deuterium.

In another embodiment, R11 to R18 are the same as or different from each other, and may be each independently hydrogen; deuterium; or a phenyl group unsubstituted or substituted with deuterium.

In one embodiment of the present application, R11 to R18 are the same as or different from each other, and may be each independently hydrogen; or deuterium.

In one embodiment of the present application, at least one of R11 to R18 may be a C6 to C60 aryl group unsubstituted or substituted with deuterium.

In one embodiment of the present application, R21 to R28 are the same as or different from each other, and each independently selected from the group consisting of hydrogen; deuterium; a halogen group; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -SiRR'R"; -P (=O) RR' ; and -NRR', or two or more groups adjacent to each other may bond to each other to form a substituted or unsubstituted C6 to C60 aromatic hydrocarbon ring or a substituted or unsubstituted C2 to C60 heteroring.

In another embodiment, R21 to R28 are the same as or different from each other, and may be each independently selected from the group consisting of hydrogen; deuterium; a substituted or unsubstituted C1 to C60 alkyl group; a substituted or unsubstituted C6 to C60 aryl group; a substituted or unsubstituted C2 to C60 heteroaryl group; - SiRR'R"; -P(=O)RR'; and -NRR'.

In another embodiment, R21 to R28 are the same as or different from each other, and may be each independently selected from the group consisting of hydrogen; deuterium; a C1 to C60 alkyl group; a C6 to C60 aryl group; a C2 to C60 heteroaryl group; -SiRR'R"; -P(=O)RR'; and -NRR'.

In another embodiment, R21 to R28 may be hydrogen; or deuterium.

In one embodiment of the present application, L may be a direct bond; a substituted or unsubstituted C6 to C60 arylene group; or a substituted or unsubstituted C2 to C60 heteroarylene group.

In another embodiment, L may be a direct bond; a substituted or unsubstituted C6 to C40 arylene group; or a substituted or unsubstituted C2 to C40 heteroarylene group.

In another embodiment, L may be a direct bond; a C6 to C40 arylene group; or a C2 to C40 heteroarylene group.

In another embodiment, L may be a direct bond; a C6 to C20 arylene group; or a C2 to C20 heteroarylene group.

In another embodiment, L may be a direct bond; a C6 to C10 arylene group; or a C2 to C10 heteroarylene group.

In another embodiment, L may be a direct bond.

In one embodiment of the present application, N-Het may be a monocyclic or polycyclic C2 to C60 heterocyclic group substituted or unsubstituted and comprising one or more Ns.

In one embodiment of the present application, N-Het may be a monocyclic or polycyclic C2 to C60 heterocyclic group substituted or unsubstituted and comprising one or more and three or less Ns.

In one embodiment of the present application, N-Het may be a monocyclic or polycyclic C2 to C60 heterocyclic group substituted or unsubstituted and comprising two or more and three or less Ns.

In one embodiment of the present application, N-Het may be a monocyclic or polycyclic C2 to C60 heterocyclic group unsubstituted or substituted with one or more substituents selected from the group consisting of a C6 to C60 aryl group and a C2 to C60 heteroaryl group, and comprising two or more and three or less Ns.

In one embodiment of the present application, Chemical Formula 1-2 may be represented by any one of the following Chemical Formulae 1-2-1 to 1-2-3.

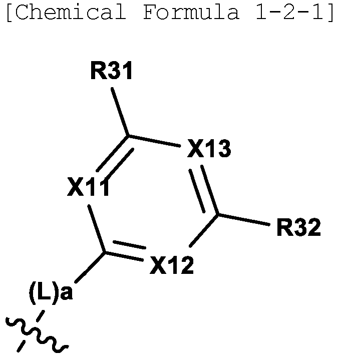

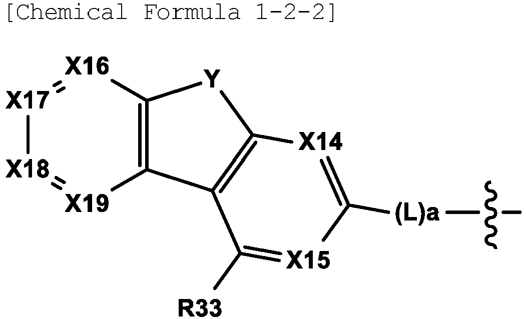

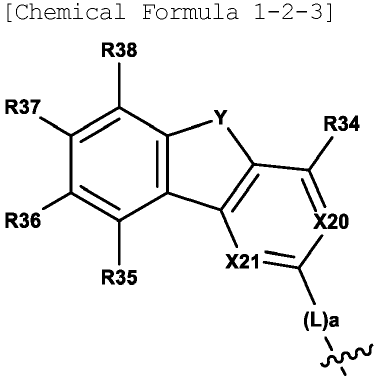

In Chemical Formulae 1-2-1 to 1-2-3,
L and a have the same definitions as in Chemical Formula 1-2, 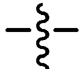 means a position linked to Chemical Formula 1,
X11 to X21 are the same as or different from each other, and each independently N; or CR40,
at least one of X11 to X13 is N, at least one of X14 to X19 is N, and at least one of X20 and X21 is N,
R40 is hydrogen; deuterium; or a substituted or unsubstituted C6 to C60 aryl group,
Y is O; or S, and
R31 to R38 are the same as or different from each other, and each independently hydrogen; deuterium; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; or a substituted or unsubstituted C2 to C60 heteroaryl group, or two or more groups adjacent to each other bond to each other to form a substituted or unsubstituted C6 to C60 aliphatic or aromatic hydrocarbon ring or a substituted or unsubstituted C2 to C60 aliphatic or aromatic heteroring.

In one embodiment of the present application, R40 may be hydrogen; deuterium; or a substituted or unsubstituted C6 to C60 aryl group.

In another embodiment, R40 may be hydrogen; deuterium; or a C6 to C60 aryl group.

In another embodiment, R40 may be hydrogen; deuterium; or a C6 to C40 aryl group.

In another embodiment, R40 may be hydrogen; or deuterium.

In one embodiment of the present application, R31 to R38 are the same as or different from each other, and each independently hydrogen; deuterium; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; or a substituted or unsubstituted C2 to C60 heteroaryl group, or two or more groups adjacent to each other may bond to each other to form a substituted or unsubstituted C6 to C60 aliphatic or aromatic hydrocarbon ring or a substituted or unsubstituted C2 to C60 aliphatic or aromatic heteroring.

In another embodiment, R31 to R38 are the same as or different from each other, and may be each independently hydrogen; deuterium; a substituted or unsubstituted C6 to C60 aryl group; or a substituted or unsubstituted C2 to C60 heteroaryl group.

In another embodiment, R31 to R38 are the same as or different from each other, and may be each independently hydrogen; deuterium; a C6 to C40 aryl group unsubstituted or substituted with deuterium, a halogen group, a C6 to C40 aryl group or a C1 to C10 alkyl group; or a C2 to C40 heteroaryl group unsubstituted or substituted with a C6 to C40 aryl group or a C1 to C10 alkyl group.

In another embodiment, R31 to R38 are the same as or different from each other, and may be each independently hydrogen; deuterium; a phenyl group unsubstituted or substituted with a naphthyl group; a biphenyl group; a naphthyl group; a dimethylfluorenyl group; a dibenzofuran group; or a dibenzothiophene group.

In one embodiment of the present application, R35 to R38 may be hydrogen; or deuterium.

In the heterocyclic compound provided in one embodiment of the present application, Chemical Formula 1 is represented by any one of the following compounds.

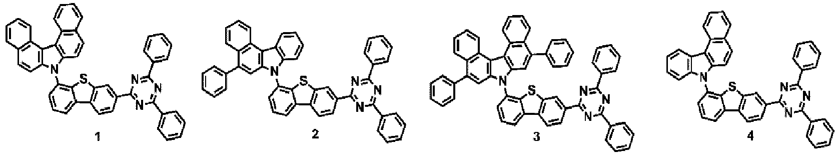

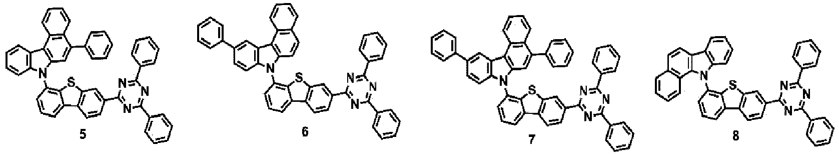

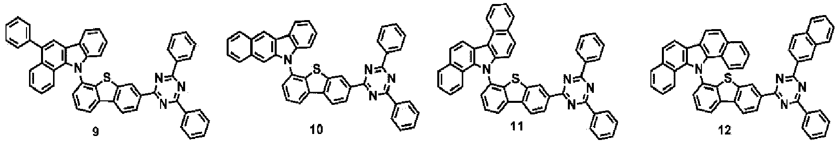

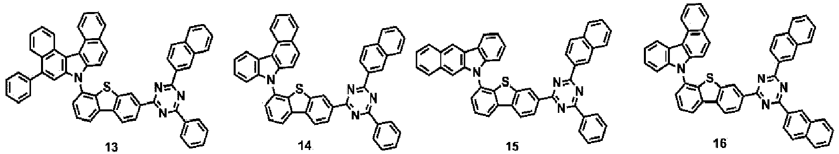

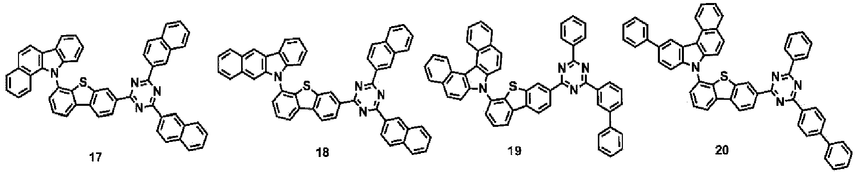

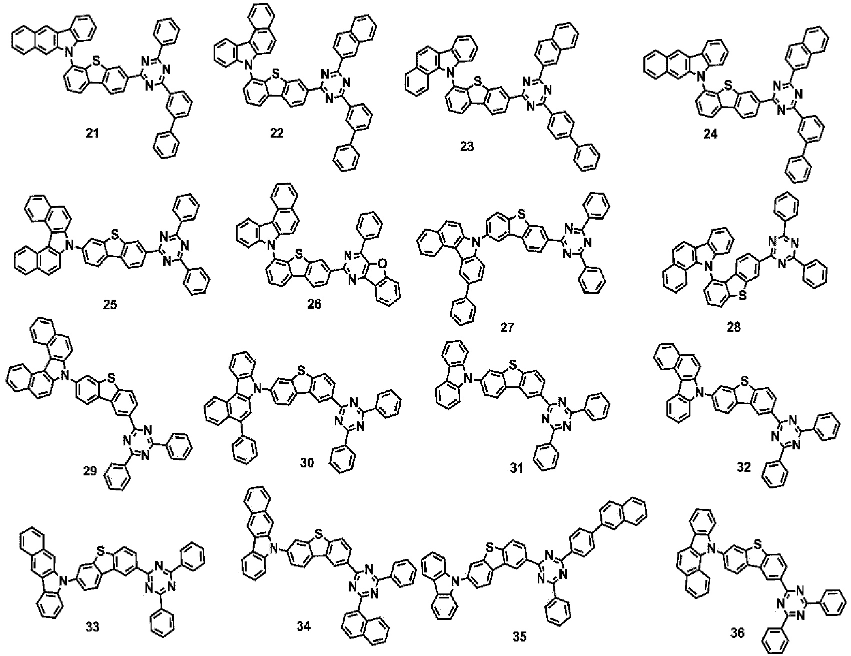

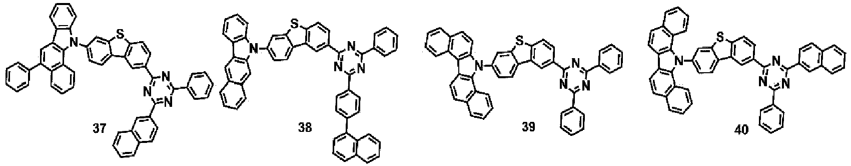

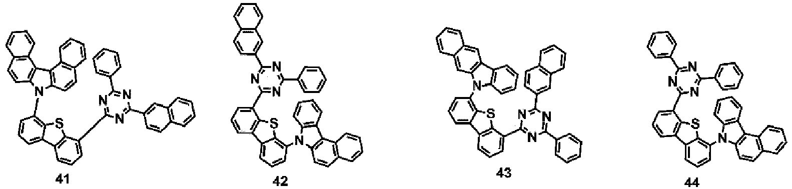

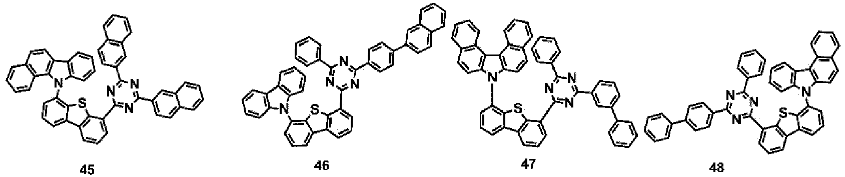

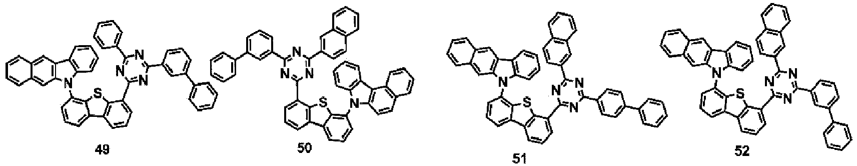

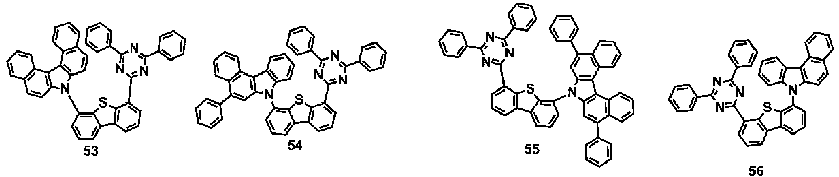

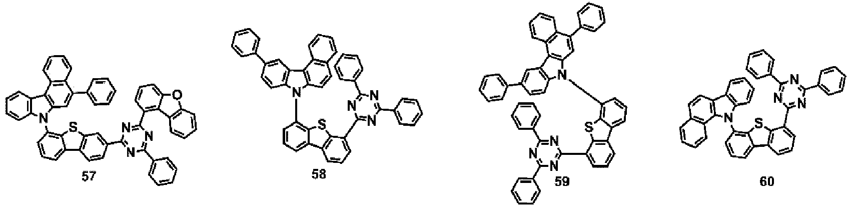

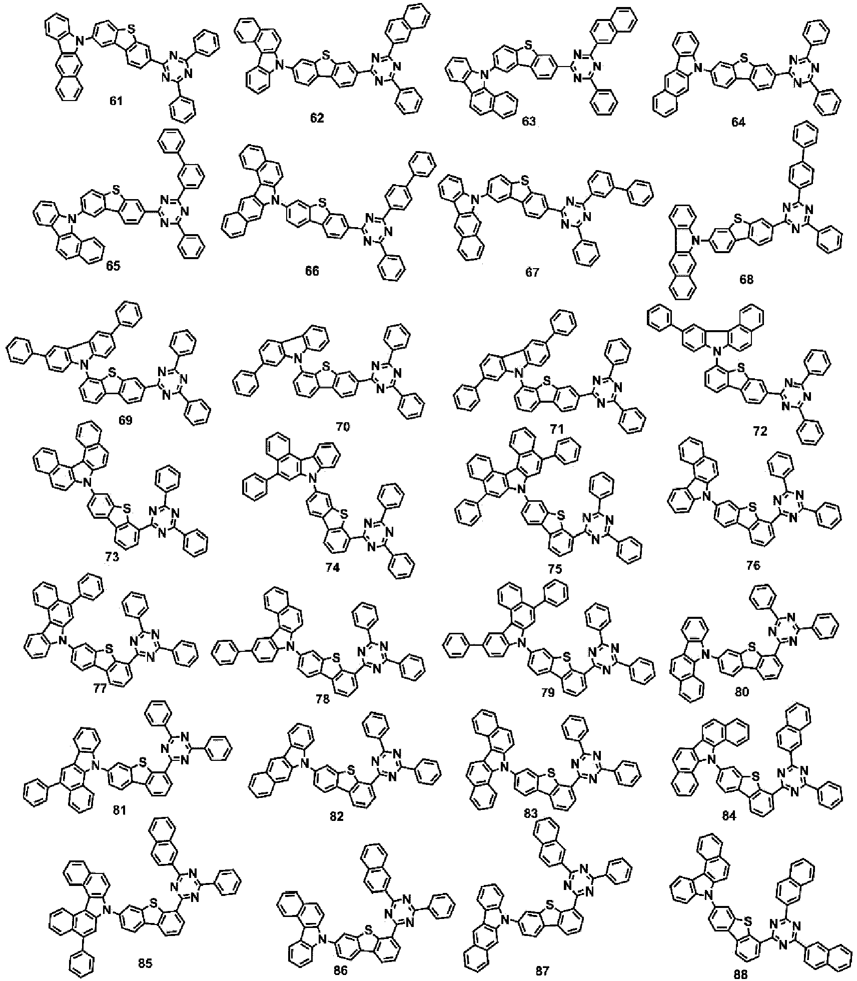

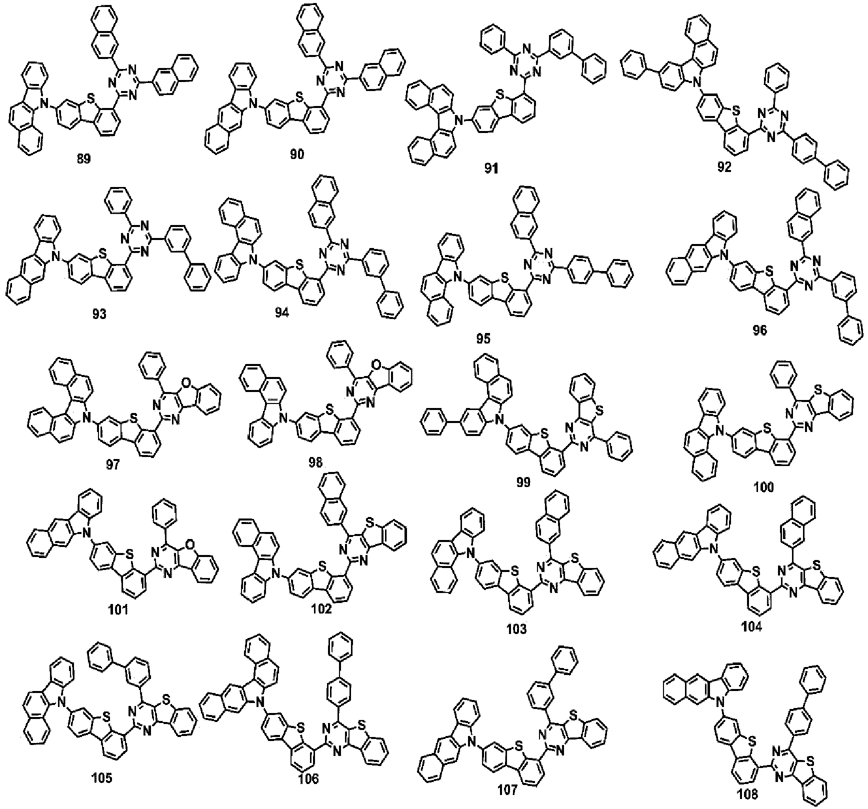

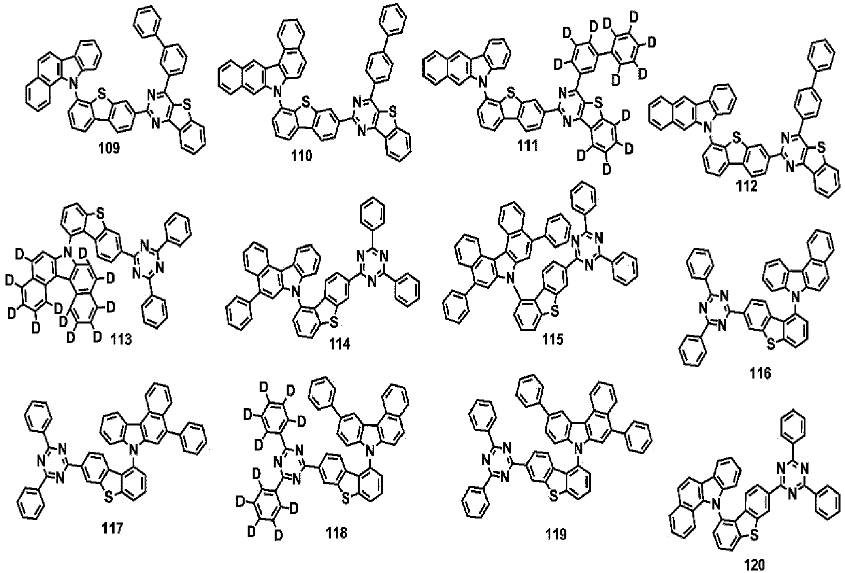

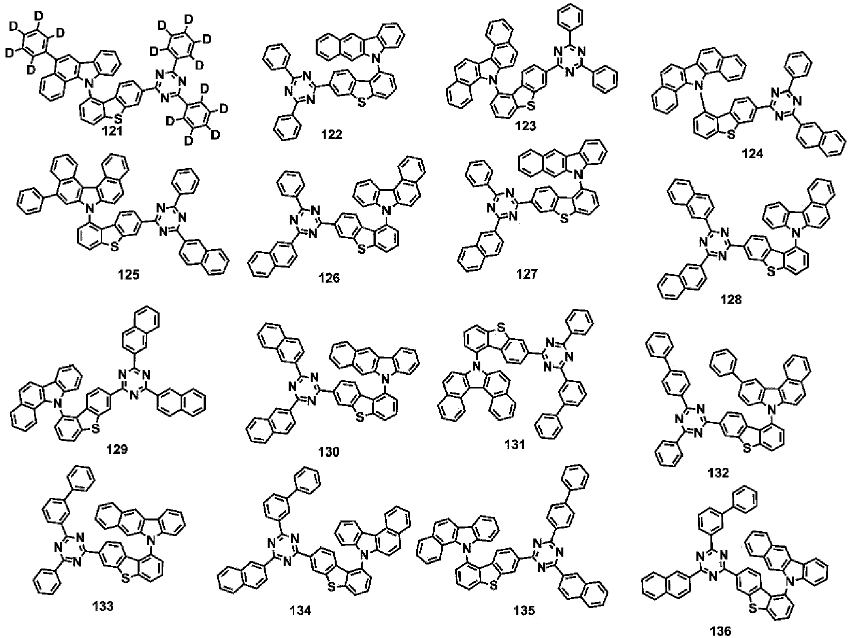

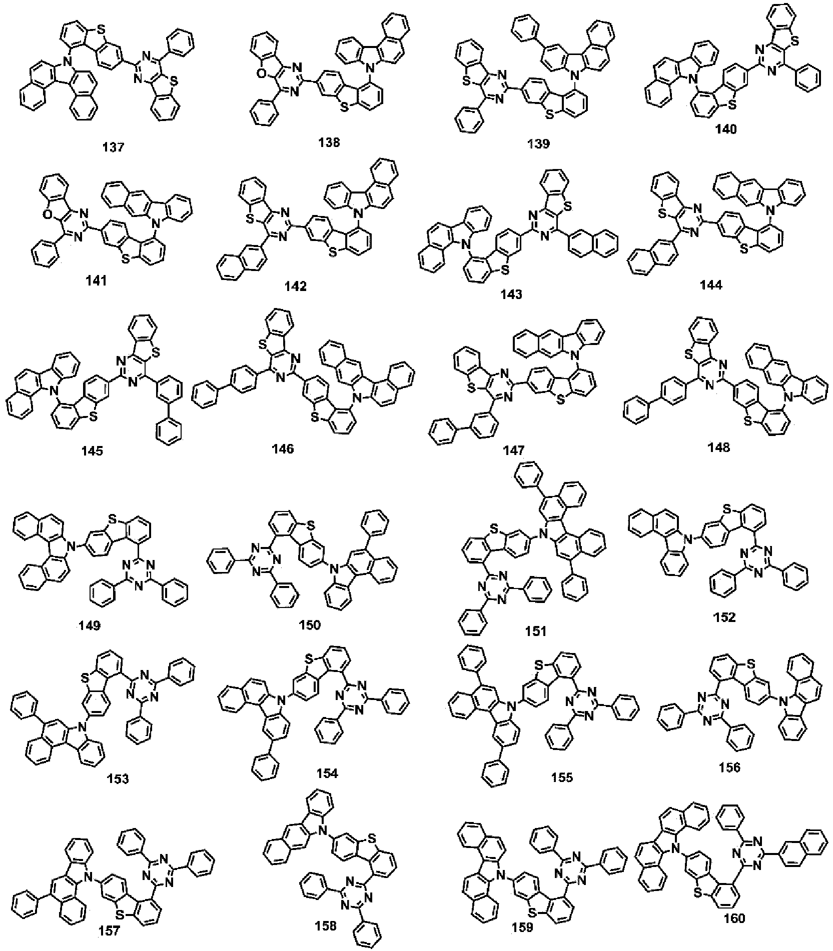

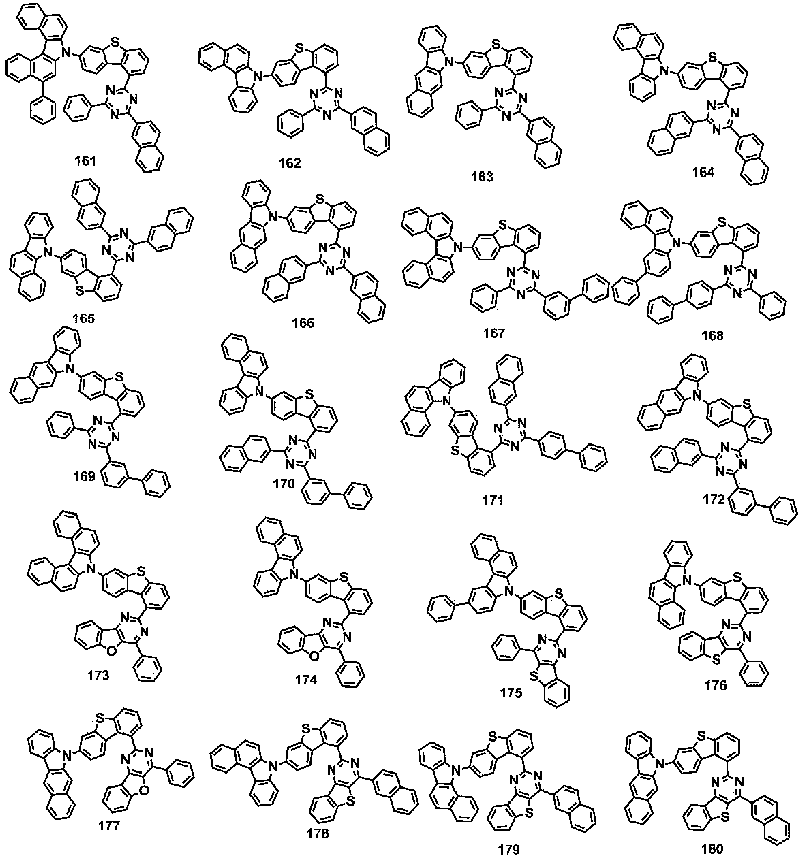

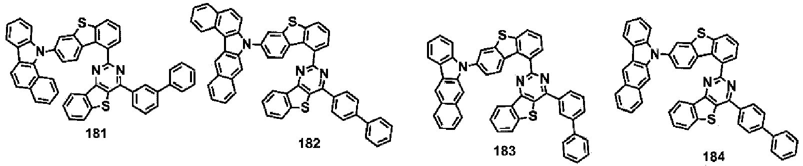

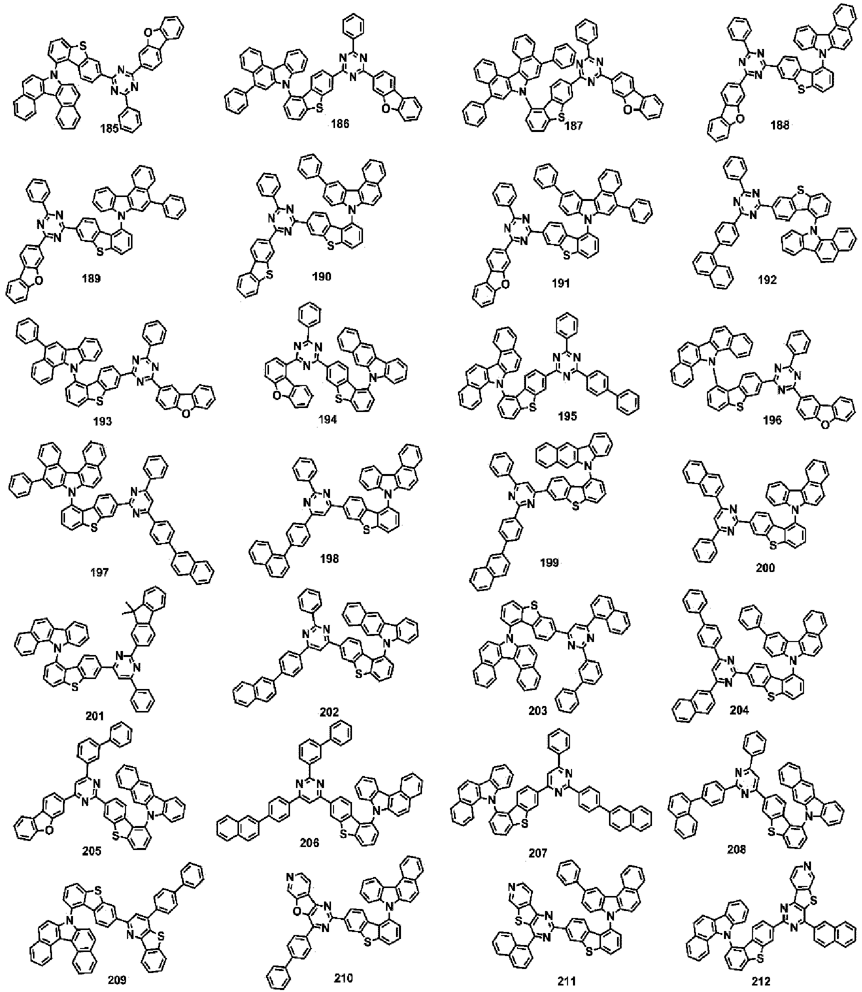

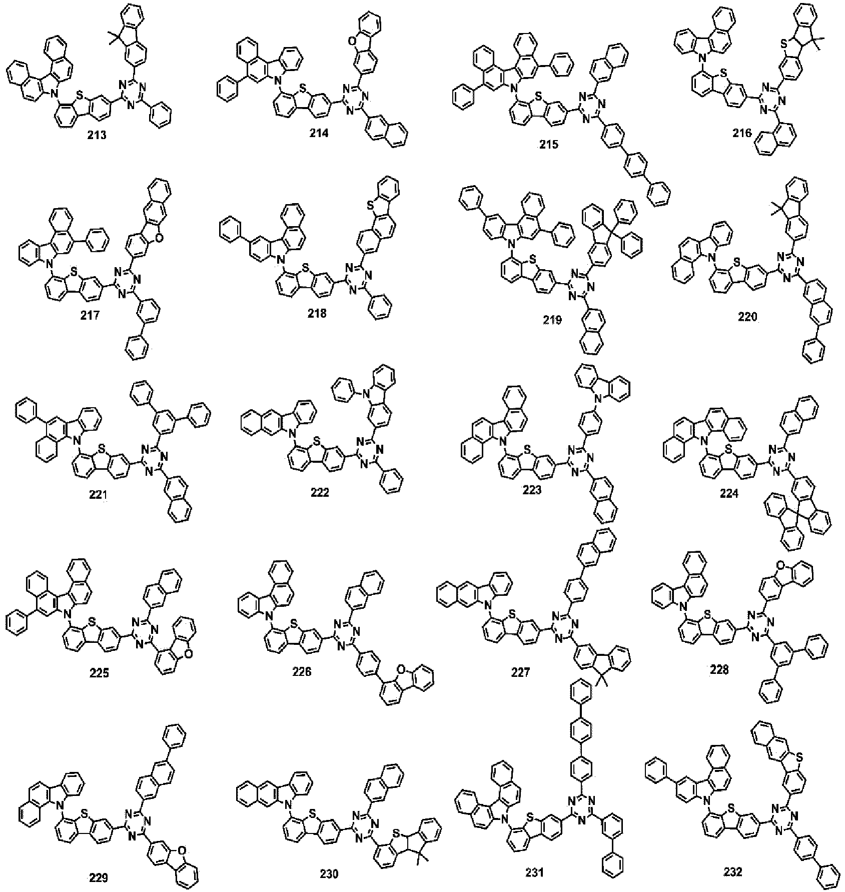

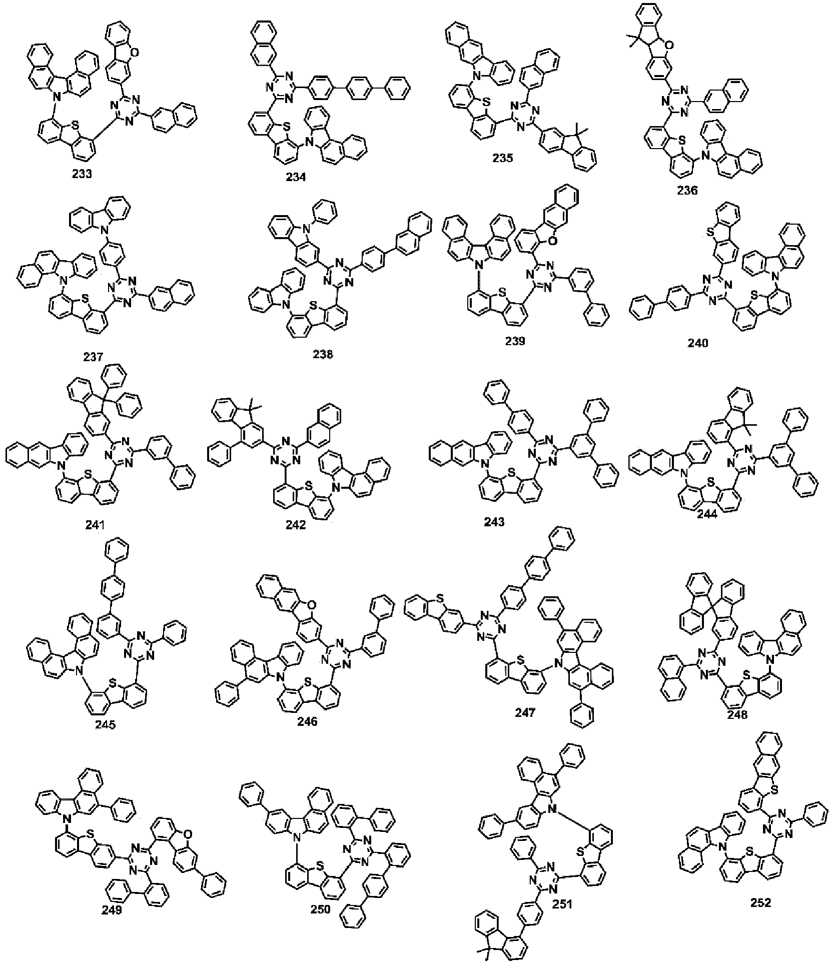

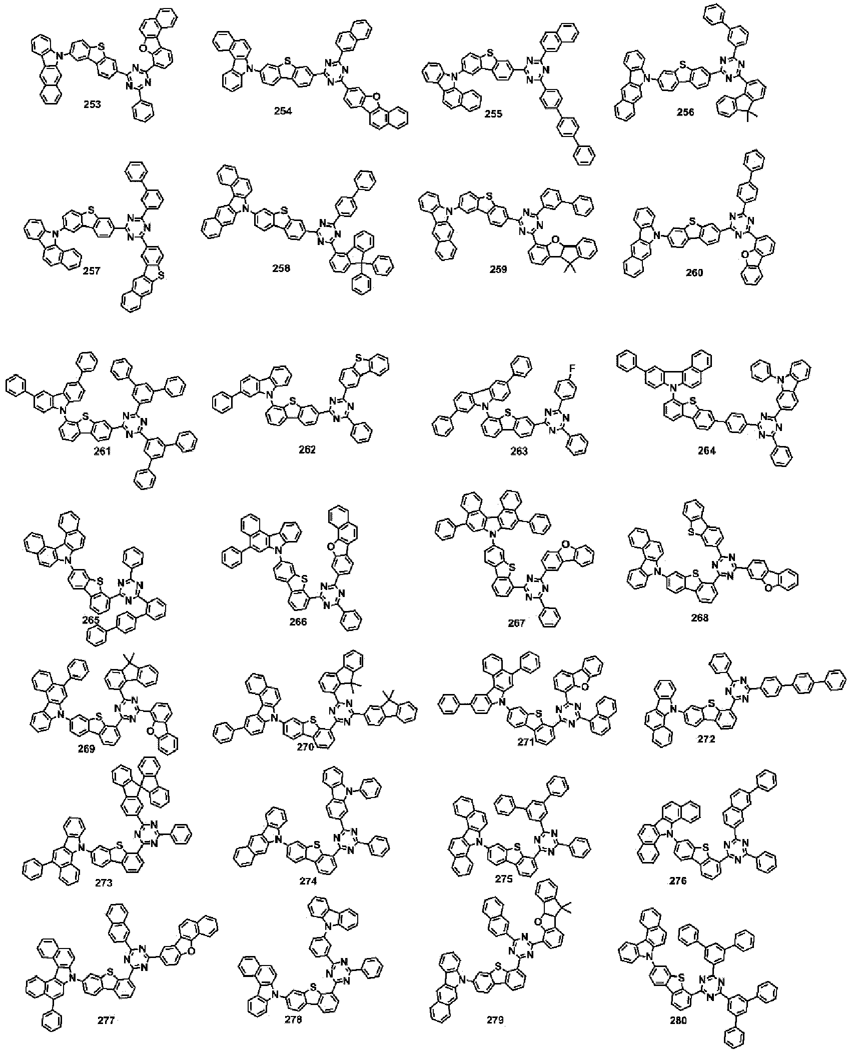

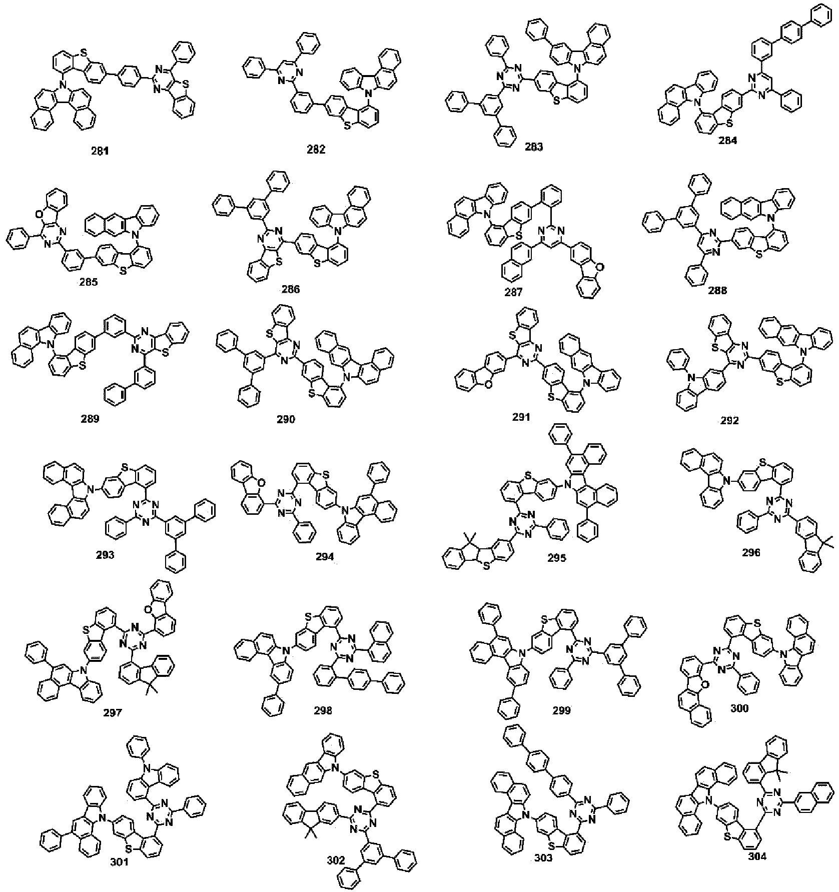

In addition, by introducing various substituents to the structure of Chemical Formula 1, compounds having unique properties of the introduced substituents may be synthesized. For example, by introducing substituents normally used as hole injection layer materials, hole transfer layer materials, light emitting layer materials, electron transfer layer materials and charge generation layer materials used for manufacturing an organic light emitting device to the core structure, materials satisfying conditions required for each organic material layer may be synthesized.

In addition, by introducing various substituents to the structure of Chemical Formula 1, the energy band gap may be finely controlled, and meanwhile, properties at interfaces between organic materials are enhanced, and material applications may become diverse.

Meanwhile, the compound has a high glass transition temperature (Tg), and has excellent thermal stability. Such an increase in the thermal stability becomes an important factor providing driving stability to a device.

In addition, one embodiment of the present application provides an organic light emitting device comprising a first electrode; a second electrode provided opposite to the first electrode; and one or more organic material layers provided between the first electrode and the second electrode, wherein one or more layers of the organic material layers comprise the heterocyclic compound represented by Chemical Formula 1.

In one embodiment of the present application, the first electrode may be an anode, and the second electrode may be a cathode.

In another embodiment, the first electrode may be a cathode, and the second electrode may be an anode.

In one embodiment of the present application, the organic light emitting device may be a blue organic light emitting device, and the heterocyclic compound according to Chemical Formula 1 may be used as a material of the blue organic light emitting device.

In one embodiment of the present application, the organic light emitting device may be a green organic light emitting device, and the heterocyclic compound represented by Chemical Formula 1 may be used as a material of the green organic light emitting device.

In one embodiment of the present application, the organic light emitting device may be a red organic light emitting device, and the heterocyclic compound represented by Chemical Formula 1 may be used as a material of the red organic light emitting device.

In one embodiment of the present application, the organic light emitting device may be a blue organic light emitting device, and the heterocyclic compound according to Chemical Formula 1 may be used as a light emitting layer material of the blue organic light emitting device.

In one embodiment of the present application, the organic light emitting device may be a green organic light emitting device, and the heterocyclic compound represented by Chemical Formula 1 may be used as a light emitting layer material of the green organic light emitting device.

In one embodiment of the present application, the organic light emitting device may be a red organic light emitting device, and the heterocyclic compound represented by Chemical Formula 1 may be used as a light emitting layer material of the red organic light emitting device.

Specific details on the heterocyclic compound represented by Chemical Formula 1 are the same as the descriptions provided above.

The organic light emitting device of the present disclosure may be manufactured using common organic light emitting device manufacturing methods and materials except that one or more organic material layers are formed using the heterocyclic compound described above.

The heterocyclic compound may be formed into an organic material layer through a solution coating method as well as a vacuum deposition method when manufacturing the organic light emitting device. Herein, the solution coating method means spin coating, dip coating, inkjet printing, screen printing, a spray method, roll coating and the like, but is not limited thereto.

The organic material layer of the organic light emitting device of the present disclosure may be formed in a single layer structure, or may also be formed in a multilayer structure in which two or more organic material layers are laminated. For example, the organic light emitting device according to one embodiment of the present disclosure may have a structure comprising a hole injection layer, a hole transfer layer, a light emitting layer, an electron transfer layer, an electron injection layer and the like as the organic material layer. However, the structure of the organic light emitting device is not limited thereto, and may comprise a smaller number of organic material layers.

In the organic light emitting device of the present disclosure, the organic material layer comprises a light emitting layer, and the light emitting layer may comprise the heterocyclic compound of Chemical Formula 1.

In the organic light emitting device of the present disclosure, the organic material layer comprises a light emitting layer, and the light emitting layer may comprise the heterocyclic compound of Chemical Formula 1 as a light emitting layer host.

In the organic light emitting device provided in one embodiment of the present application, the organic material layer comprising the heterocyclic compound represented by Chemical Formula 1 further comprises a heterocyclic compound represented by the following Chemical Formula 2. 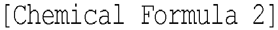 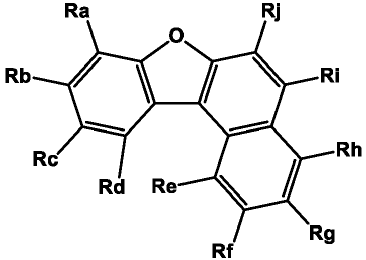 In Chemical Formula 2,
Ra to Rj are the same as or different from each other, and each independently selected from the group consisting of hydrogen; deuterium; a halogen group; -CN; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -SiR101R102R103; - P(=O)R101R102; and -NR101R102, or two or more groups adjacent to each other bond to each other to form a substituted or unsubstituted C6 to C60 aromatic hydrocarbon ring or a substituted or unsubstituted C2 to C60 heteroring,
R101, R102 and R103 are the same as or different from each other, and each independently hydrogen; deuterium; -CN; a substituted or unsubstituted C1 to C60 alkyl group; a substituted or unsubstituted C6 to C60 aryl group; or a substituted or unsubstituted C2 to C60 heteroaryl group, and
at least one of Ra to Rj is a substituted or unsubstituted C2 to C60 heteroaryl group.

Effects of more superior efficiency and lifetime are obtained when comprising the heterocyclic compound of Chemical Formula 1 and the heterocyclic compound of Chemical Formula 2 in the organic material layer of the organic light emitting device. Such results may lead to a forecast that an exciplex phenomenon occurs when comprising the two compounds at the same time.

The exciplex phenomenon is a phenomenon of releasing energy having sizes of a donor (p-host) HOMO level and an acceptor (n-host) LUMO level due to electron exchanges between two molecules. When the exciplex phenomenon occurs between two molecules, reverse intersystem crossing (RISC) occurs, and as a result, internal quantum efficiency of fluorescence may increase up to 100%. When a donor (p-host) having a favorable hole transfer ability and an acceptor (n-host) having a favorable electron transfer ability are used as a host of a light emitting layer, holes are injected to the p-host and electrons are injected to the n-host, and therefore, a driving voltage may be lowered, which resultantly helps with enhancement in the lifetime.

In one embodiment of the present application, Chemical Formula 2 may be represented by the following Chemical Formula 2-1. 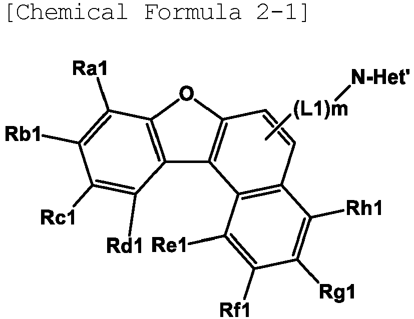 In Chemical Formula 2-1,
Ra1 to Rh1 are each independently selected from the group consisting of hydrogen; a substituted or unsubstituted C1 to C60 alkyl group; a substituted or unsubstituted C6 to C60 aryl group; and a substituted or unsubstituted C2 to C60 heteroaryl group,
L1 is a direct bond; a substituted or unsubstituted arylene group; or a substituted or unsubstituted heteroarylene group,
N-Het' is a monocyclic or polycyclic C2 to C60 heterocyclic group substituted or unsubstituted and comprising one or more Ns, and
m is an integer of 0 to 4.

In one embodiment of the present application, L1 of Chemical Formula 2-1 has the same definition as L of Chemical Formula 1-2.

In one embodiment of the present application, N-Het' may be a monocyclic or polycyclic C2 to C60 heterocyclic group substituted or unsubstituted and comprising one or more Ns.

In one embodiment of the present application, N-Het' may be a monocyclic or polycyclic C2 to C60 heterocyclic group substituted or unsubstituted and comprising two or more and three or less Ns.

In one embodiment of the present application, N-Het' may be a monocyclic or polycyclic C2 to C60 heterocyclic group unsubstituted or substituted with one or more substituents selected from the group consisting of a C6 to C60 aryl group and a C2 to C60 heteroaryl group, and comprising two or more and three or less Ns.

In one embodiment of the present application, N-Het' may be a triazine group unsubstituted or substituted with one or more substituents selected from the group consisting of a C6 to C60 aryl group and a C2 to C60 heteroaryl group; or a benzo[4,5]thieno[3,2-d]pyrimidine group unsubstituted or substituted with one or more substituents selected from the group consisting of a C6 to C60 aryl group and a C2 to C60 heteroaryl group.

In one embodiment of the present application, N-Het' may be a triazine group unsubstituted or substituted with one or more substituents selected from the group consisting of a phenyl group, a biphenyl group and a dibenzofuran group; or a benzo[4,5]thieno[3,2-d]pyrimidine group unsubstituted or substituted with a phenyl group.

In addition, another embodiment of the present application provides a composition for an organic material layer of an organic light emitting device, the composition comprising the heterocyclic compound represented by Chemical Formula 1 and the heterocyclic compound represented by Chemical Formula 2.

Specific descriptions on the heterocyclic compound represented by Chemical Formula 1 and the heterocyclic compound represented by Chemical Formula 2 are the same as the descriptions provided above.

In the composition, the heterocyclic compound represented by Chemical Formula 1:the heterocyclic compound represented by Chemical Formula 2 may have a weight ratio of 1:10 to 10:1, 1:8 to 8:1, 1:5 to 5:1 or 1:2 to 2:1, however, the weight ratio is not limited thereto.

The composition may be used when forming an organic material of an organic light emitting device, and may be more preferably used when forming a host of a light emitting layer.

In one embodiment of the present application, the organic material layer comprises the heterocyclic compound represented by Chemical Formula 1 and the heterocyclic compound represented by Chemical Formula 2, and a phosphorescent dopant may be used therewith.

In one embodiment of the present application, the organic material layer comprises the heterocyclic compound represented by Chemical Formula 1 and the heterocyclic compound represented by Chemical Formula 2, and an iridium-based dopant may be used therewith.

As a material of the phosphorescent dopant, those known in the art may be used.

For example, phosphorescent dopant materials represented by LL'MX', LL'L"M, LMX'X", L2MX' and L3M may be used, however, the scope of the present disclosure is not limited to these examples.

Herein, L, L', L", X' and X" are a bidentate ligand different from each other, and M is a metal forming an octahedral complex.

M may comprise iridium, platinum, osmium and the like.

L, L' and L" are an anionic bidentate ligand coordinated to M as the iridium-based dopant by sp2 carbon and heteroatom, and X' and X" may function to trap electrons or holes. Nonlimiting examples of L may comprise 2-(1-naphthyl)benzoxazole, (2-phenylbenzoxazole), (2-phenylbenzothiazole), (2-phenylbenzothiazole), (7,8-benzoquinoline), (thiophene group pyrizine), phenylpyridine, benzothiophene group pyrizine, 3-methoxy-2-phenylpyridine, thiophene group pyrizine, tolylpyridine and the like. Nonlimiting examples of X' and X" may comprise acetylacetonate (acac), hexafluoroacetylacetonate, salicylidene, picolinate, 8-hydroxyquinolinate and the like.

More specific examples thereof are described below, however, the phosphorescent dopant is not limited to these examples.

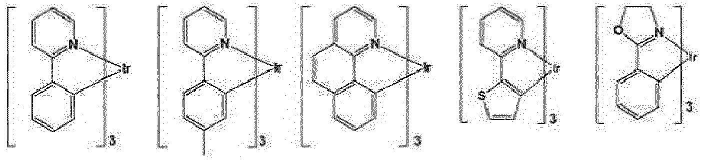

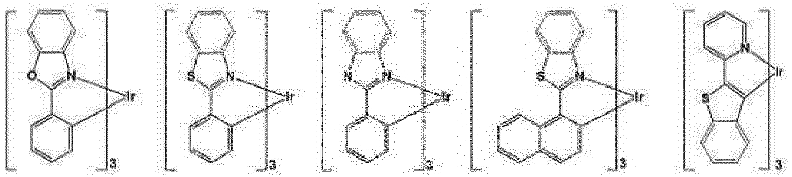

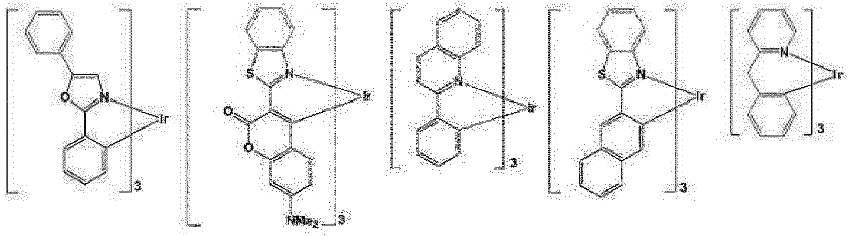

In one embodiment of the present application, as the iridium-based dopant, Ir(ppy)₃ may be used as a green phosphorescent dopant.

In one embodiment of the present application, a content of the dopant may be from 1% to 15%, preferably from 3% to 10% and more preferably from 5% to 10% based on the whole light emitting layer.

In the organic light emitting device of the present disclosure, the organic material layer comprises an electron injection layer or an electron transfer layer, and the electron injection layer or the electron transfer layer may comprise the heterocyclic compound represented by Chemical Formula 1.

In another organic light emitting device, the organic material layer comprises an electron blocking layer or a hole blocking layer, and the electron blocking layer or the hole blocking layer may comprise the heterocyclic compound represented by Chemical Formula 1.

In another organic light emitting device, the organic material layer comprises an electron transfer layer, a light emitting layer or a hole blocking layer, and the electron transfer layer, the light emitting layer or the hole blocking layer may comprise the heterocyclic compound represented by Chemical Formula 1.

The organic light emitting device of the present disclosure may further comprise one, two or more layers selected from the group consisting of a light emitting layer, a hole injection layer, a hole transfer layer, an electron injection layer, an electron transfer layer, an electron blocking layer and a hole blocking layer.

FIG. 1 to FIG. 3 illustrate a lamination order of electrodes and organic material layers of an organic light emitting device according to one embodiment of the present application. However, the scope of the present application is not limited to these diagrams, and structures of organic light emitting devices known in the art may also be used in the present application.

FIG. 1 illustrates an organic light emitting device in which an anode (200), an organic material layer (300) and a cathode (400) are consecutively laminated on a substrate (100). However, the structure is not limited to such a structure, and as illustrated in FIG. 2, an organic light emitting device in which a cathode, an organic material layer and an anode are consecutively laminated on a substrate may also be obtained.

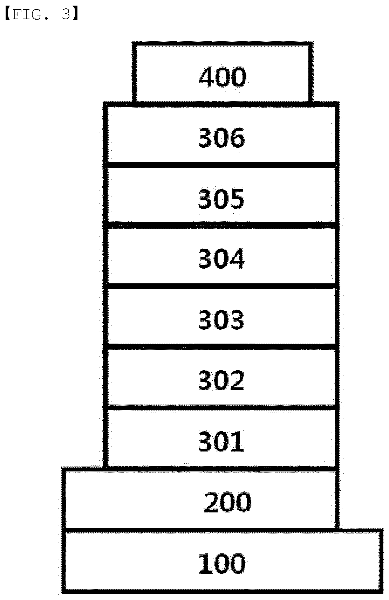

FIG. 3 illustrates a case of the organic material layer being a multilayer. The organic light emitting device according to FIG. 3 comprises a hole injection layer (301), a hole transfer layer (302), a light emitting layer (303), a hole blocking layer (304), an electron transfer layer (305) and an electron injection layer (306). However, the scope of the present application is not limited to such a lamination structure, and as necessary, layers other than the light emitting layer may not be included, and other necessary functional layers may be further added.

One embodiment of the present application provides a method for manufacturing an organic light emitting device, the method comprising preparing a substrate; forming a first electrode on the substrate; forming one or more organic material layers on the first electrode; and forming a second electrode on the organic material layer, wherein the forming of organic material layers comprises forming one or more organic material layers using the composition for an organic material layer according to one embodiment of the present application.

In the method for manufacturing an organic light emitting device provided in one embodiment of the present application, the forming of organic material layers is forming the heterocyclic compound of Chemical Formula 1 and the heterocyclic compound of Chemical Formula 2 using a thermal vacuum deposition method after pre-mixing.

The pre-mixing means first mixing the heterocyclic compound of Chemical Formula 1 and the heterocyclic compound of Chemical Formula 2 in one source of supply before depositing on the organic material layer.

The premixed material may be referred to as the composition for an organic material layer according to one embodiment of the present application.

The organic material layer comprising Chemical Formula 1 may further comprise other materials as necessary.

The organic material layer comprising both Chemical Formula 1 and Chemical Formula 2 may further comprise other materials as necessary.

In the organic light emitting device according to one embodiment of the present application, materials other than the compound of Chemical Formula 1 or Chemical Formula 2 are illustrated below, however, these are for illustrative purposes only and not for limiting the scope of the present application, and may be replaced by materials known in the art.

As the anode material, materials having relatively large work function may be used, and transparent conductive oxides, metals, conductive polymers or the like may be used. Specific examples of the anode material comprise metals such as vanadium, chromium, copper, zinc and gold, or alloys thereof; metal oxides such as zinc oxide, indium oxide, indium tin oxide (ITO) and indium zinc oxide (IZO); combinations of metals and oxides such as ZnO:Al or SnO₂:Sb; conductive polymers such as poly(3-methylthiophene), poly[3,4-(ethylene-1,2-dioxy)thiophene] (PEDOT), polypyrrole and polyaniline, and the like, but are not limited thereto.

As the cathode material, materials having relatively small work function may be used, and metals, metal oxides, conductive polymers or the like may be used. Specific examples of the cathode material comprise metals such as magnesium, calcium, sodium, potassium, titanium, indium, yttrium, lithium, gadolinium, aluminum, silver, tin and lead, or alloys thereof; multilayer structure materials such as LiF/Al or LiO₂/Al, and the like, but are not limited thereto.

As the hole injection material, known hole injection materials may be used, and for example, phthalocyanine compounds such as copper phthalocyanine disclosed in US Patent No. 4,356,429, or starburst-type amine derivatives such as tris(4-carbazoyl-9-ylphenyl)amine (TCTA), 4,4',4"-tri[phenyl(m-tolyl)amino]triphenylamine (m-MTDATA) or 1,3,5-tris[4-(3-methylphenylphenylamino)phenyl]benzene (m-MTDAPB) described in the literature [Advanced Material, 6, p.677 (1994)], polyaniline/dodecylbenzene sulfonic acid, poly(3,4-ethylenedioxythiophene)/poly(4-styrenesulfonate), polyaniline/camphor sulfonic acid or polyaniline/poly(4-styrene-sulfonate) that are conductive polymers having solubility, and the like, may be used.

As the hole transfer material, pyrazoline derivatives, arylamine-based derivatives, stilbene derivatives, triphenyldiamine derivatives and the like may be used, and low molecular or high molecular materials may also be used.

As the electron transfer material, metal complexes of oxadiazole derivatives, anthraquinodimethane and derivatives thereof, benzoquinone and derivatives thereof, naphthoquinone and derivatives thereof, anthraquinone and derivatives thereof, tetracyanoanthraquinodimethane and derivatives thereof, fluorenone derivatives, diphenyldicyanoethylene and derivatives thereof, diphenoquinone derivatives, 8-hydroxyquinoline and derivatives thereof, and the like, may be used, and high molecular materials may also be used as well as low molecular materials.

As examples of the electron injection material, LiF is typically used in the art, however, the present application is not limited thereto.

As the light emitting material, red, green or blue light emitting materials may be used, and as necessary, two or more light emitting materials may be mixed and used. Herein, two or more light emitting materials may be used by being deposited as individual sources of supply or by being premixed and deposited as one source of supply. In addition, fluorescent materials may also be used as the light emitting material, however, phosphorescent materials may also be used. As the light emitting material, materials emitting light by bonding electrons and holes injected from an anode and a cathode, respectively, may be used alone, however, materials having a host material and a dopant material involving in light emission together may also be used.

When mixing light emitting material hosts, same series hosts may be mixed, or different series hosts may be mixed. For example, any two or more types of materials among n-type host materials or p-type host materials may be selected and used as a host material of a light emitting layer.

The organic light emitting device according to one embodiment of the present application may be a top-emission type, a bottom-emission type or a dual-emission type depending on the materials used.

The heterocyclic compound according to one embodiment of the present application may also be used in an organic electronic device comprising an organic solar cell, an organic photo conductor, an organic transistor and the like under a similar principle used in the organic light emitting device.

Hereinafter, the present specification will be described in more detail with reference to examples, however, these are for illustrative purposes only, and the scope of the present application is not limited thereto.

### <Preparation Example>

### <Preparation Example 1> Preparation of Compound 178-P

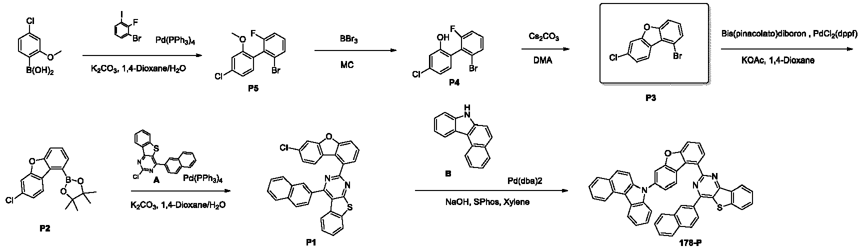

### Preparation of Compound P5

In a one neck round bottom flask (one neck r.b.f), a mixture of (4-chloro-2-(methylthio)phenyl)boronic acid (30.98 g, 166.21 mmol), 1-bromo-2-iodo-3-fluorobenzene (50 g, 151.10 mmol), tetrakis(triphenylphosphine)palladium(0) (8.73 g, 7.56 mmol), potassium carbonate (41.77 g, 302.2 mmol) and 1,4-dioxane/H₂O (500 ml/150 ml) was refluxed at 120°C.

The result was extracted with dichloromethane, dried with MgSO₄, and column purified to obtain Compound P5 (34.8 g, 73%).

### Preparation of Compound P4

In a one neck round bottom flask (one neck r.b.f), a mixture of (2'-bromo-4-chloro-6'-fluoro-[1,1'-biphenyl]-2-yl) (methyl) sulfane (34.8 g, 110.28 mmol) and MC (348 ml) was cooled to 0°C, and after adding BBr₃ (20.9 ml, 220.56 mmol) dropwise thereto, the temperature was raised to room temperature, and the mixture was stirred for 2 hours.

The reaction was terminated using distilled water, and the result was extracted with dichloromethane and dried with MgSO₄. The result was silica gel filtered, and then concentrated to obtain Compound P4 (33.25 g, 100%).

### Preparation of Compound P3

In a one neck round bottom flask (one neck r.b.f), a mixture of 2'-bromo-4-chloro-6'-fluoro-[1,1'-biphenyl]-2-thiol (33.25 g, 110.27 mmol), Cs₂CO₃ (71.86 g, 220.54 mmol) and dimethylacetamide (330 ml) was stirred at 150°C. The result was cooled and then filtered, and the solvent of the filtrate was silica gel filtered to obtain Compound P3 (24.84 g, 80%).

### Preparation of Compound P2

A mixture of 1-bromo-7-chlorodibenzo[b,d]thiophene (24.84 g, 88.23 mmol), bis(pinacolato)diboron (44.81 g, 176.46 mmol), potassium acetate (25.98 g, 264.69 mmol), PdCl₂(dppf) (3.23 g, 4.41 mmol) and 1,4-dioxane (240 ml) was refluxed at 140°C.

The result was extracted with dichloromethane, concentrated, and then silica gel filtered to obtain Compound P2 (34.79 g, over yield).

### Preparation of Compound P1

In a one neck round bottom flask (one neck r.b.f), a mixture of 2-(7-chlorodibenzo[b,d]thiophen-1-yl)-4,4,5,5-tetramethyl-1,3,2-dioxaborolane (34.79 g, 105.87 mmol), 2-chloro-4-(naphthalen-2-yl)benzo[4,5]thieno[3,2-d]pyrimidine (40.39 g, 116.46 mmol), tetrakis(triphenylphosphine)palladium(0) (6.12 g, 5.29 mmol), potassium carbonate (29.26 g, 211.74 mmol) and 1,4-dioxane/water (340 ml/102 ml) was refluxed for 3 hours at 120°C. The result was filtered at room temperature, and then washed with distilled water and acetone to obtain Compound P1 (52.68 g, 97%).

### Preparation of Compound 178-P

In a one neck round bottom flask (one neck r.b.f), a mixture of 2-(7-chlorodibenzo[b,d]thiophen-1-yl)-4-(naphthalen-2-yl)benzo[4,5]thieno[2,3-d]pyrimidine (52.68 g, 102.70 mmol), 7H-benzo[c]carbazole (24.54 g, 112.97 mmol), Pd(dba)₂ (5.95 g, 5.14 mmol), sodium hydroxide (8.22 g, 205.4 mmol), SPhos (6.32 g, 15.41 mmol) and xylene (445 ml) was refluxed for 8 hours at 180°C.

The result was cooled and then filtered, and washed with distilled water and acetone to obtain Compound 178-P (59.14 g, 83%) .

Target compounds were prepared in the same manner as in Preparation Example 1 except that Intermediate A of the following Table 1 was used instead of 2-chloro-4-(naphthalen-2-yl)benzo[4,5]thieno[3,2-d]pyrimidine, and Intermediate B of the following Table 1 was used instead of 7H-benzo[c]carbazole.

**[Table 1]**

| Compo und | A | B | C | Yield (1-1-1(C)-c to C) |
|---|---|---|---|---|
| 150 | 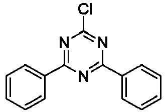 | 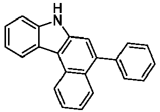 | 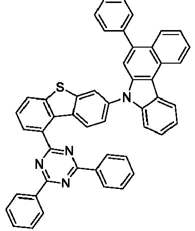 | 71% |
| 161 | 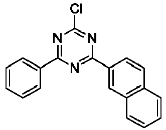 | 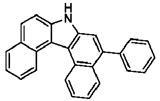 | 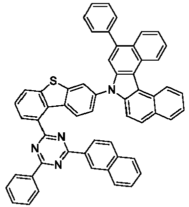 | 74% |
| 163 | 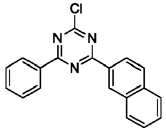 | 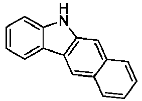 | 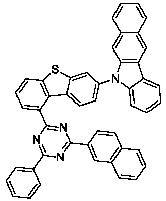 | 67% |
| 168 | 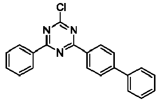 | 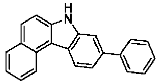 | 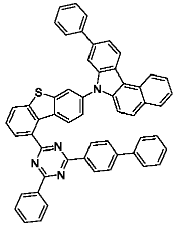 | 66% |
| 172 | 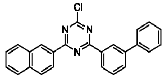 | 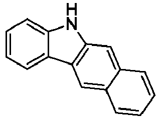 | 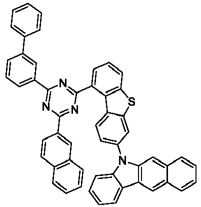 | 70% |
| 176 | 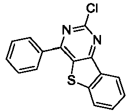 | 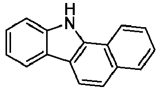 | 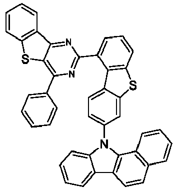 | 64% |
| 181 | 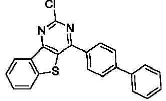 | 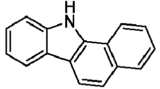 | 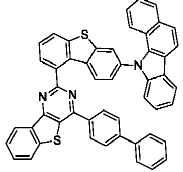 | 72% |
| 184 | 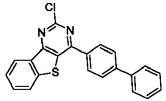 | 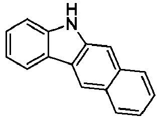 | 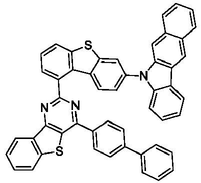 | 76% |

### <Preparation Example 2> Preparation of Compound 70-P

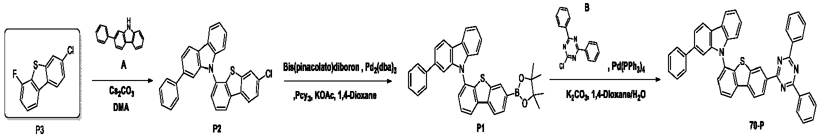

Compound 70-P (10.89 g, 68%) was synthesized in the same manner as in Preparation Example 1 except that 3-chloro-6-fluorodibenzo[b,d]thiophene was used instead of 1-bromo-7-chlorodibenzo[b,d]thiophene.

Target compounds were synthesized in the same manner as in Preparation Example 2 except that Intermediate A of the following Table 2 was used instead of the compound represented by A, and Intermediate B of the following Table 2 was used instead of the compound represented by B.

**[Table 2]**

| Compo und | A | B | C | Yield |
|---|---|---|---|---|
| 3 | 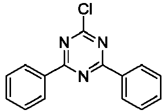 | 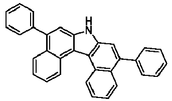 | 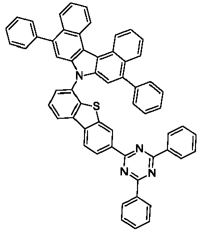 | 75% |
| 17 | 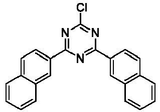 | 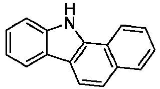 | 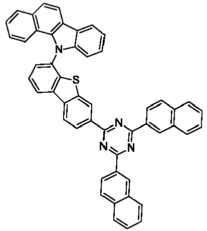 | 69 |
| 23 | 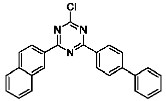 | 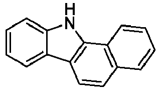 | 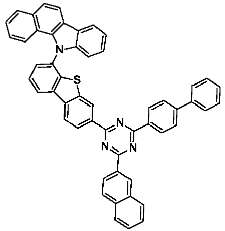 | 78% |
| 26 | 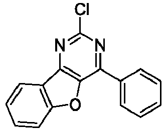 | 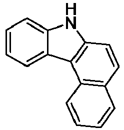 | 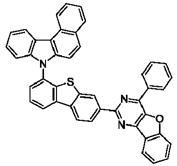 | 59% |
| 69 | 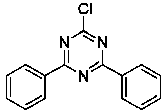 | 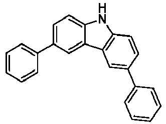 | 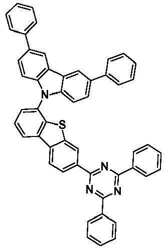 | 65% |

The heterocyclic compounds of Chemical Formula 1 other than the compounds described in Preparation Example 1, Preparation Example 2, Table 1 and Table 2 were also prepared in the same manner as described in the preparation examples described above.

The following Table 3 and Table 4 show 1H NMR data and FD-MS data of the synthesized compounds, and through the following data, syntheses of the target compounds are identified.

**[Table 3]**

| **NO** | **¹H NMR (CDCl₃, 300 Mz)** |
|---|---|
| **1** | 8.54 (d, 2H), 8.45 (d, 1H), 8.28 (d, 4H), 8.16 (m, 2H), 8.11-8.08 (m, 2H), 7.96-7.94 (m, 2H), 7.82 (d, 1H), 7.67-7.63 (m, 6H), 7.52-7.41 (m, 8H). |
| **27** | 8.54 (d, 1H), 8.28 (d, 4H), 8.18-8.08 (m, 4H), 8.00-7.94 (m, 3H), 7.82 (d, 1H), 7.77 (s, 1H), 7.67-7.63 (m, 4H), 7.52-7.41 (m, 12H) |
| **33** | 8.55 (d, 1H), 8.28 (d, 4H), 8.16 (t, 2H), 8.05-8.00 (m, 3H), 7.94 (d, 1H), 7.86-7.80 (m, 2H), 7.67 (d, 2H), 7.55-7.25 (m, 11H) |
| **49** | 8.57 (d, 1H), 8.45-8.41 (m, 2H), 8.28-8.16 (m, 5H), 7.94 (d, 1H), 7.80 (d, 1H), 7.67-7.25 (m, 18H) |
| **65** | 8.55 (m, 1H), 8.28-8.24 (m, 3H), 8.16-8.08 (m, 4H), 7.98-7.94 (m, 3H), 7.82 (d, 1H), 7.70-7.67 (m, 3H), 7.57-7.25 (m, 11H) |
| **80** | 8.55-8.51 (m, 2H), 8.41 (d, 1H), 8.28 (d, 4H), 8.16 (m, 1H), 8.12 (d, 1H), 8.05-8.02 (m, 2H), 7.94 (d, 1H), 7.80 (d, 1H), 7.67-7.25 (m, 13H) |
| **92** | 8.55-8.51 (m, 2H), 8.41 (d, 1H), 8.28 (d, 4H), 8.16 (m, 1H), 8.12 (d, 1H), 8.05-8.02 (m, 2H), 7.94 (d, 1H), 7.80 (d, 1H), 7.67-7.25 (m, 13H) |
| **109** | 8.54 (d, 1H), 8.41 (d, 1H), 8.28 (d, 4H), 8.18-8.16 (m, 2H), 8.05-8.00 (m, 3H), 7.85 (d, 2H), 7.80-7.77 (m, 2H), 7.67-7.41 (m, 19H), 7.25 (d, 2H) |
| **118** | 8.54 (d, 1H), 8.28 (d, 4H), 8.18-8.00 (m, 4H), 8.11 (d, 1H), 7.98 (d, 1H), 7.82 (d, 1H), 7.77 (s, 1H), 7.67-7.63 (m, 4H), 7.52-7.41 (m, 13H) |
| **129** | 9.09 (s, 2H), 8.55-8.49 (m, 4H), 8.16-7.92 (m, 12H), 7.82 (d, 1H), 7.67 (d, 2H), 7.59-7.49 (m, 9H), 7.33 (m, 1H), 7.25 (m, 1H) |
| **140** | 9.09 (s, 2H), 8.55-8.49 (m, 4H), 8.16-7.92 (m, 12H), 7.82 (d, 1H), 7.67 (d, 2H), 7.59-7.49 (m, 9H), 7.33 (m, 1H), 7.25 (m, 1H) |
| **156** | 8.55-8.51 (m, 2H), 8.28 (d, 4H), 8.16-8.02 (m, 4H), 7.94 (d, 2H), 7.82 (d, 1H), 7.67 (d, 2H), 7.57-7.25 (m, 11H) |
| **162** | 9.09 (s, 2H), 8.55-8.49 (m, 4H), 8.16-7.92 (m, 12H), 7.82 (d, 1H), 7.67 (d, 2H), 7.59-7.49 (m, 9H), 7.33 (m, 1H), 7.25 (m, 1H) |
| **171** | 9.09 (s, 1H), 8.55-8.49 (m, 3H), 8.16-7.82 (m, 12H), 7.67-7.25 (m, 16H) |
| **178** | 8.55-8.54 (m, 2H), 8.34 (s, 1H), 8.16 (m, 1H), 8.05-7.87 (m, 11H), 7.67-7.49 (m, 10H), 7.33 (m, 1H), 7.25 (m, 1H) |
| **180** | 8.55 (d, 1H), 8.34 (s, 1H), 8.16 (m, 2H), 8.05-7.82 (m, 11H), 7.67-7.49 (m, 9H), 7.40 (s, 1H), 7.33-7.25 (m, 2H) |
| 182 | 8.54 (m, 1H), 8.30 (d, 1H), 8.16 (m, 2H), 8.05-7.94 (m, 5H), 7.85-7.82 (m, 3H), 7.67-7.40 (m, 17H) |

**[Table 4]**

| **Comp ound** | **FD-MS** | **Compo und** | **FD-MS** |
|---|---|---|---|
| **1** | m/z=680.20 (C₄₇H₂₈N₄S=680.82) | **3** | m/z=832.27 (C₅₉H₃₆N₄S=833.01) |
| **5** | m/z=706.22 (C₄₉H₃₀N₄S=706.85) | **7** | m/z=782.25 (C₅₅H₃₄N₄S=782.95) |
| **9** | m/z=706.22 (C₄₉H₃₀N₄S=706.85) | **11** | m/z=680.20 (C₄₇H₂₈N₄S=680.82) |
| **13** | m/z=806.25 (C₅₇H₃₄N₄S=806.97) | **15** | m/z=680.20 (C₄₇H₂₈N₄S=680.82) |
| **17** | m/z=730.22 (C₅₁H₃₀N₄S=730.88) | **19** | m/z=756.23 (C₅₃H₃₂N₄S=756.91) |
| **21** | m/z=706.22 (C₄₉H₃₀N₄S=706.85) | **23** | m/z=756.23 (C₅₃H₃₂N₄S=756.91) |
| **25** | m/z=680.20 (C₄₇H₂₈N₄S=680.82) | **27** | m/z=706.22 (C₄₉H₃₀N₄S=706.85) |
| **29** | m/z=680.20 (C₄₇H₂₈N₄S=680.82) | **31** | m/z=580.17 (C₃₉H₂₄N₄S=580.70) |
| **33** | m/z=630.19 (C₄₃H₂₆N₄S=630.76) | **35** | m/z=706.22 (C₄₉H₃₀N₄S=706.85) |
| **37** | m/z=756.23 (C₅₃H₃₂N₄S=756.91) | **39** | m/z=680.20 (C₄₇H₂₈N₄S=680.82) |
| **41** | m/z=730.22 (C₅₁H₃₀N₄S=730.88) | **43** | m/z=680.20 (C₄₇H₂₈N₄S=680.82) |
| **45** | m/z=730.22 (C₅₁H₃₀N₄S=730.88) | **47** | m/z=756.23 (C₅₃H₃₂N₄S=756.91) |
| **49** | m/z=706.22 (C₄₉H₃₀N₄S=706.85) | **51** | m/z=756.23 (C₅₃H₃₂N₄S=756.91) |
| **53** | m/z=680.20 (C₄₇H₂₈N₄S=680.82) | **55** | m/z=832.27 (C₅₉H₃₆N₄S=833.01) |
| **57** | m/z=796.23 (C₅₅H₃₂N₄OS=796.93) | **59** | m/z=782.25 (C₅₅H₃₄N₄S=782.95) |
| **61** | m/z=630.19 (C₄₃H₂₆N₄S=630.76) | **63** | m/z=680.20 (C₄₇H₂₈N₄S=680.82) |
| **65** | m/z=706.22 (C₄₉H₃₀N₄S=706.85) | **67** | m/z=706.22 (C₄₉H₃₀N₄S=706.85) |
| **69** | m/z=732.23 (C₅₁H₃₂N₄S=732.89) | **71** | m/z=732.23 (C₅₁H₃₂N₄S=732.89) |
| **73** | m/z=680.20 (C₄₇H₂₈N₄S=680.82) | **75** | m/z=832.27 (C₅₉H₃₆N₄S=833.01) |
| **77** | m/z=706.22 (C₄₉H₃₀N₄S=706.85) | **79** | m/z=782.25 (C₅₅H₃₄N₄S=782.95) |
| **81** | m/z=706.22 (C₄₉H₃₀N₄S=706.85) | **83** | m/z=680.20 (C₄₇H₂₈N₄S=680.82) |
| **85** | m/z=806.25 (C₅₇H₃₄N₄S=806.97) | **87** | m/z=680.20 (C₄₇H₂₈N₄S=680.82) |
| **89** | m/z=730.22 (C₅₁H₃₀N₄S=730.88) | **91** | m/z=756.23 (C₅₃H₃₂N₄S=756.91) |
| **93** | m/z=706.22 (C₄₉H₃₀N₄S=706.85) | **95** | m/z=756.23 (C₅₃H₃₂N₄S=756.91) |
| **97** | m/z=693.19 (C₄₈H₂₇N₃OS=693.81) | **99** | m/z=735.18 (C₅₀H₂₉N₃S₂=735.92) |
| **101** | m/z=643.17 (C₄₄H₂₅N₃OS=643.75) | **103** | m/z=709.16 (C₄₈H₂₇N₃S₂=709.88) |
| **105** | m/z=735.18 (C₅₀H₂₉N₃S₂=735.92) | **107** | m/z=735.18 (C₅₀H₂₉N₃S₂=735.92) |
| **109** | m/z=735.18 (C₅₀H₂₉N₃S₂=735.92) | **111** | m/z=735.18 (C₅₀H₂₉N₃S₂=735.92) |
| **113** | m/z=680.20 (C₄₇H₂₈N₄S=680.82) | **115** | m/z=832.27 (C₅₉H₃₆N₄S=833.01) |
| **117** | m/z=706.22 (C₄₉H₃₀N₄S=706.85) | **119** | m/z=782.25 (C₅₅H₃₄N₄S=782.95) |
| **121** | m/z=706.22 (C₄₉H₃₀N₄S=706.85) | **123** | m/z=680.20 (C₄₇H₂₈N₄S=680.82) |
| **125** | m/z=806.25 (C₅₇H₃₄N₄S=806.97) | **127** | m/z=680.20 (C₄₇H₂₈N₄S=680.82) |
| **129** | m/z=730.22 (C₅₁H₃₀N₄S=730.88) | **131** | m/z=756.23 (C₅₃H₃₂N₄S=756.91) |
| **133** | m/z=706.22 (C₄₉H₃₀N₄S=706.85) | **135** | m/z=756.23 (C₅₃H₃₂N₄S=756.91) |
| **137** | m/z=709.16 (C₄₈H₂₇N₃S₂=709.88) | **139** | m/z=735.18 (C₅₀H₂₉N₃S₂=735.92) |
| **141** | m/z=643.17 (C₄₄H₂₅N₃OS=643.75) | **143** | m/z=709.16 (C₄₈H₂₇N₃S₂=709.88) |
| **145** | m/z=735.18 (C₅₀H₂₉N₃S₂=735.92) | **147** | m/z=735.18 (C₅₀H₂₉N₃S₂=735.92) |
| **149** | m/z=680.20 (C₄₇H₂₈N₄S=680.82) | **151** | m/z=832.27 (C₅₉H₃₆N₄S=833.01) |
| **153** | m/z=706.22 (C₄₉H₃₀N₄S=706.85) | **155** | m/z=782.25 (C₅₅H₃₄N₄S=782.95) |
| **157** | m/z=706.22 (C₄₉H₃₀N₄S=706.85) | **159** | m/z=680.20 (C₄₇H₂₈N₄S=680.82) |
| **161** | m/z=806.25 (C₅₇H₃₄N₄S=806.97) | **163** | m/z=680.20 (C₄₇H₂₈N₄S=680.82) |
| **165** | m/z=730.22 (C₅₁H₃₀N₄S=730.88) | **167** | m/z=756.23 (C₅₃H₃₂N₄S=756.91) |
| **169** | m/z=706.22 (C₄₉H₃₀N₄S=706.85) | **171** | m/z=756.23 (C₅₃H₃₂N₄S=756.91) |
| **173** | m/z=693.19 (C₄₈H₂₇N₃OS=693.81) | **175** | m/z=735.18 (C₅₀H₂₉N₃S₂=735.92) |
| **177** | m/z=643.17 (C₄₄H₂₅N₃OS=643.75) | **179** | m/z=709.16 (C₄₈H₂₇N₃S₂=709.88) |
| **181** | m/z=735.18 (C₅₀H₂₉N₃S₂=735.92) | **183** | m/z=735.18 (C₅₀H₂₉N₃S₂=735.92) |
| **185** | m/z=770.21 (C₅₃H₃₀N₄OS=770.90) | **187** | m/z=922.28 (C₆₅H₃₈N₄OS=923.09) |
| **189** | m/z=796.23 (C₅₅H₃₂N₄OS=796.93) | **191** | m/z=872.26 (C₆₁H₃₆N₄OS=873.03) |
| **193** | m/z=796.23 (C₅₅H₃₂N₄OS=796.93) | **195** | m/z=756.23 (C₅₃H₃₂N₄S=756.91) |
| **197** | m/z=881.29 (C₆₄H₃₉N₃S=882.08) | **199** | m/z=755.24 (C₅₄H₃₂N₃S=755.92) |
| 201 | m/z=745.26 (C₅₃H₃₄N₃S=745.93) | 203 | m/z=805.26 (C₅₈H₃₄N₃S=805.98) |
| 205 | m/z=795.23 (C₅₆H₃₂N₃OS=795.95) | 207 | m/z=755.24 (C₅₄H₃₂N₃S=755.92) |
| 209 | m/z=784.20 (C₅₅H₃₁N₂S₂=784.99) | 211 | m/z=786.19 (C₅₅H₃₁N₂S₂=786.96) |

### <Experimental Example 1>-Manufacture of Organic Light Emitting Device

### 1) Manufacture of Organic Light Emitting Device

A glass substrate on which indium tin oxide (ITO) was coated as a thin film to a thickness of 1,500 Å was cleaned with distilled water ultrasonic waves. After the cleaning with distilled water was finished, the substrate was ultrasonic cleaned with solvents such as acetone, methanol and isopropyl alcohol, then dried, and UVO treatment was conducted for 5 minutes using UV in a UV cleaner. After that, the substrate was transferred to a plasma cleaner (PT), and after conducting plasma treatment under vacuum for ITO work function and residual film removal, the substrate was transferred to a thermal deposition apparatus for organic deposition.

On the transparent ITO electrode (anode), the following 2-TNATA (4,4',4"-tris[2-naphthyl(phenyl)amino]triphenylamine) and the following NPB (N,N'-di(1-naphthyl)-N,N'-diphenyl-(1,1'-biphenyl)-4,4'-diamine) were formed respectively as a hole injection layer and a hole transfer layer, which are common layers.

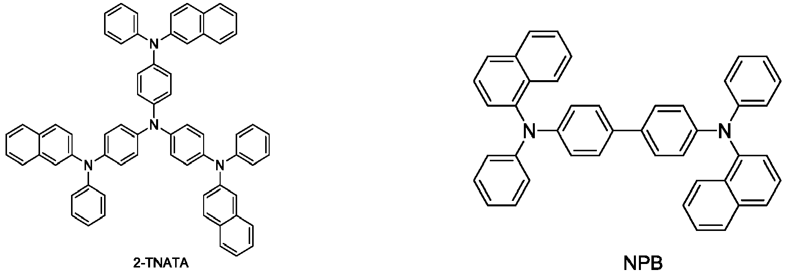

A light emitting layer was thermal vacuum deposited thereon as follows. The light emitting layer was deposited to 500 Å by depositing one, or two types of compounds described in the following Table 5 in one source of supply as a red host, and doping the following (piq)₂(Ir) (acac) to the host by 3% as a red phosphorescent dopant. After that, the following BCP was deposited to 60 Å as a hole blocking layer, and Alq₃ was deposited to 200 Å thereon as an electron transfer layer. Lastly, an electron injection layer was formed on the electron transfer layer by depositing lithium fluoride (LiF) to a thickness of 10 Å, and then a cathode was formed on the electron injection layer by depositing an aluminum (Al) cathode to a thickness of 1,200 Å, and as a result, an organic electroluminescent device was manufactured.

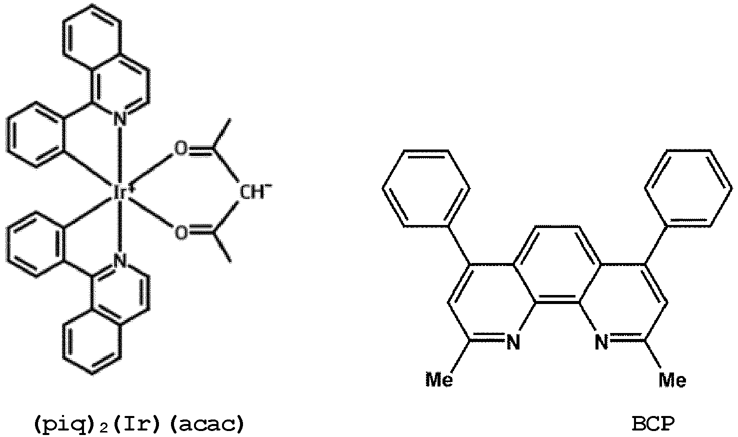

Meanwhile, all the organic compounds required to manufacture the OLED were vacuum sublimation purified under 10⁻⁸ torr to 10⁻⁶ torr for each material to be used in the OLED (organic light emitting device) manufacture.

### 2) Driving Voltage and Light Emission Efficiency of Organic Electroluminescent Device

For each of the organic electroluminescent devices manufactured as above, electroluminescent (EL) properties were measured using M7000 manufactured by McScience Inc., and with the measurement results, T90 was measured when standard luminance was 6,000 cd/m² through a lifetime measurement system (M6000) manufactured by McScience Inc.. Properties of the organic electroluminescent devices of the present disclosure are as shown in the following Table 5.

**[Table 5]**

| | Compound | | Driving Voltage (V) | Efficiency (cd/A) | Color Coordinate (x, y) | Lifetime (T₉₀) |
|---|---|---|---|---|---|---|
| Comparative Example 1 | A | | 5.43 | 18.8 | (0.681, 0.319) | 44 |
| Comparative Example 2 | B | | 5.36 | 19.5 | (0.682, 0.316) | 29 |
| Comparative Example 3 | C | | 5.29 | 20.1 | (0.683, 0.315) | 49 |
| Comparative Example 4 | D | | 4.95 | 15.9 | (0.681, 0.318) | 52 |
| Comparative Example 5 | E | | 4.89 | 16.8 | (0.680, 0.319) | 65 |
| Example 1 | 5 | | 3.84 | 18.8 | (0.681, 0.318) | 109 |
| Example 2 | 7 | | 3.87 | 19.5 | (0.680, 0.319) | 165 |
| Example 3 | 18 | | 3.79 | 25.9 | (0.680, 0.319) | 159 |
| Example 4 | 18:H | 3:1 | 3.79 | 37.8 | (0.682, 0.316) | 189 |
| Example 5 | | 1:1 | 3.52 | 36.9 | (0.683, 0.315) | 183 |
| Example 6 | | 1:3 | 2.91 | 35.7 | (0.681, 0.318) | 190 |
| Example 7 | 26 | | 4.14 | 37.8 | (0.680, 0.319) | 89 |
| Example 8 | 68 | | 3.87 | 23.2 | (0.681, 0.318) | 137 |
| Example 9 | 68:I | 3:1 | 3.92 | 31.2 | (0.682, 0.316) | 195 |
| Example 10 | | 1:1 | 3.98 | 30.8 | (0.683, 0.315) | 196 |
| Example 11 | | 1:3 | 4.01 | 29.8 | (0.681, 0.318) | 189 |
| Example 12 | 111 | | 3.79 | 22.8 | (0.682, 0.316) | 119 |
| Example 13 | 27 | | 3.59 | 26.8 | (0.683, 0.315) | 79 |
| Example 14 | 129 | | 4.06 | 22.0 | (0.681, 0.318) | 144 |
| Example 15 | 129:G | 3:1 | 3.95 | 30.3 | (0.680, 0.319) | 198 |
| Example 16 | | 1:1 | 4.06 | 29.0 | (0.680, 0.319) | 192 |
| Example 17 | | 1:3 | 4.09 | 27.9 | (0.680, 0.319) | 197 |
| Example 18 | 34 | | 3.99 | 25.9 | (0.680, 0.319) | 138 |
| Example 19 | 34:H | 1:1 | 3.95 | 26.8 | (0.681, 0.318) | 181 |
| Example 20 | 40 | | 3.88 | 27.8 | (0.680, 0.319) | 148 |
| Example 21 | 49 | | 3.90 | 28.8 | (0.680, 0.319) | 146 |
| Example 22 | 53 | | 3.89 | 29.5 | (0.680, 0.319) | 176 |
| Example 23 | 53:I | 1:1 | 3.90 | 32.9 | (0.681, 0.318) | 190 |
| Example 24 | 77 | | 3.78 | 28.2 | (0.681, 0.318) | 202 |
| Example 25 | 84 | | 4.11 | 36.2 | (0.681, 0.318) | 151 |
| Example 26 | 99 | | 4.01 | 37.6 | (0.681, 0.318) | 121 |
| Example 27 | 105 | | 3.92 | 33.8 | (0.681, 0.318) | 228 |
| Example 28 | 65 | | 4.21 | 28.2 | (0.681, 0.318) | 244 |
| Example 29 | 65:I | 3:1 | 3.98 | 29.3 | (0.681, 0.318) | 185 |
| Example 30 | | 1:1 | 4.09 | 28.0 | (0.680, 0.319) | 196 |
| Example 31 | | 1:3 | 4.12 | 26.9 | (0.680, 0.319) | 179 |
| Example 32 | 150 | | 3.69 | 40. | (0.681, 0.318) | 189 |
| Example 33 | 159 | | 3.63 | 39.5 | (0.681, 0.318) | 170 |
| Example 34 | 178 | | 3.80 | 27.9 | (0.682, 0.316) | 178 |
| Example 35 | 162:F | 1:1 | 3.73 | 36.9 | (0.681, 0.318) | 206 |
| Example 36 | 162:G | 1:1 | 3.69 | 39.5 | (0.681, 0.318) | 227 |
| Example 37 | 162:H | 1:1 | 3.62 | 40.2 | (0.681, 0.318) | 231 |
| Example 38 | 179 | | 3.88 | 26.5 | (0.683, 0.315) | 221 |
| Example 39 | 180 | | 3.90 | 23.8 | (0.681, 0.318) | 103 |
| Example 40 | 183 | | 3.98 | 26.2 | (0.680, 0.319) | 121 |
| Comparative Example 6 | A:F=1:1 | | 4.92 | 21.2 | (0.680, 0.319) | 133 |
| Comparative Example 7 | D:F=1:1 | | 5.01 | 19.9 | (0.681, 0.318) | 140 |
| Comparative Example 8 | D:H=1:1 | | 4.89 | 20.8 | (0.679, 0.320) | 162 |
| Example 44 | 168:H | 7:1 | 3.49 | 48.9 | (0.682, 0.316) | 218 |
| Example 45 | | 5:1 | 3.49 | 49.8 | (0.681, 0.318) | 230 |
| Example 46 | | 3:1 | 3.52 | 48.5 | (0.681, 0.318) | 241 |
| Example 47 | | 1:1 | 3.60 | 46.3 | (0.682, 0.316) | 252 |

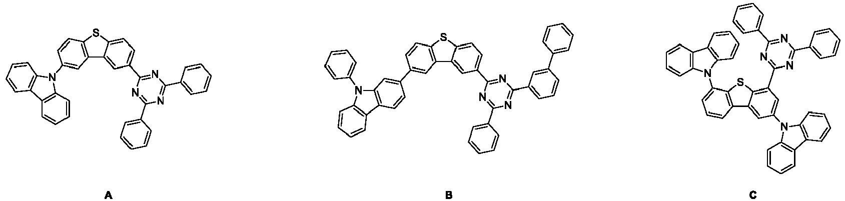

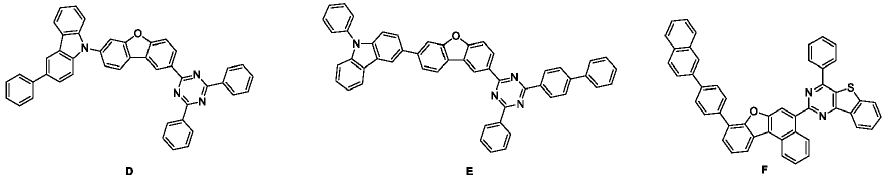

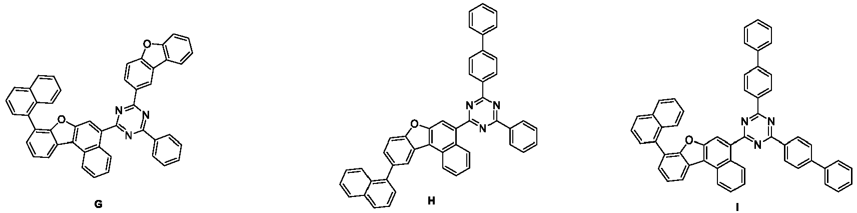

As seen from Table 1, the compounds of Chemical Formula 1 provide proper energy level and thermal stability to the device by one side benzene ring of the dibenzothiophene structure being substituted with a carbazole derivative having HT properties (Chemical Formula 1-1) and the other side benzene ring being substituted with a substituent having ET properties (Chemical Formula 1-2), and it was identified that an organic light emitting device having improved lifetime, driving stability and efficiency was able to be manufactured using the compounds of Chemical Formula 1.

Lifetime, efficiency and driving voltage were different in the properties depending on the structure, and as a result of identifying electron cloud of the compounds for each structure through molecular calculation, it was seen that HOMO and LUMO tended to be well separated when the B4 position is substituted with ET (Chemical Formula 1-2) and the A2 position is substituted with HT (Chemical Formula 1-1) in the dibenzothiophene of Chemical Formula 1. When other positions are substituted, it was identified that electron cloud of HOMO and LUMO was mostly spread, and properties of lifetime, efficiency and driving voltage were not favorable compared to in the molecules in which HOMO and LUMO are well separated.

In addition, HOMO and LUMO data for the heterocyclic compound of Chemical Formula 1 of the present application are as shown in the following Table 6 and Table 7.

**[Table 6]**

| | Compound of Present Applicatio n | HOMO | | Eg | LUMO | |
|---|---|---|---|---|---|---|
| Example 1-1 | 4 | -5.60 | 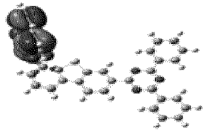 | 3.14 | -2.46 | 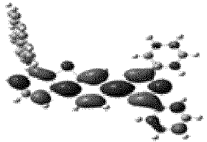 |
| Example 1-2 | 25 | -5.46 | 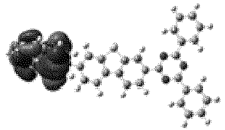 | 2.93 | -2.50 | 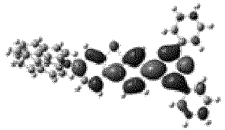 |
| Example 1-3 | 27 | -5.63 | 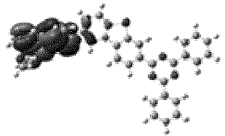 | 3.13 | -2.50 | 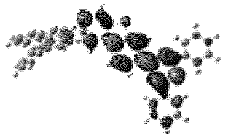 |
| Example 1-4 | 120 | -5.56 | 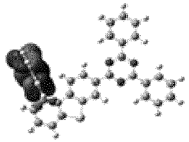 | 3.15 | -2.42 | 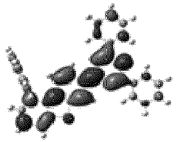 |
| Example 1-5 | 35 | -5.73 | 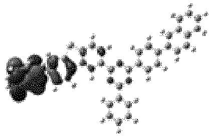 | 3.35 | -2.38 | 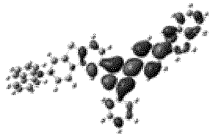 |
| Example 1-6 | 41 | -5.43 | 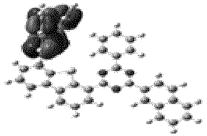 | 3.08 | -2.35 | 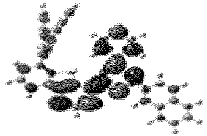 |
| Example 1-7 | 57 | -5.55 | 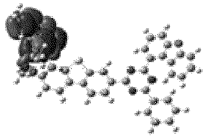 | 3.05 | -2.50 | 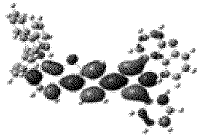 |
| Example 1-8 | 73 | -5.42 | 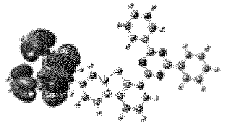 | 2.97 | -2.44 | 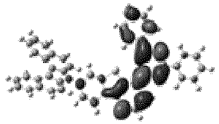 |
| Example 1-9 | 149 | -5.41 | 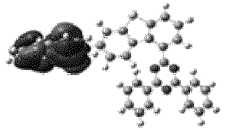 | 3.03 | -2.38 | 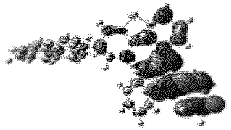 |

**[Table 7]**

| | S₁ | T₁ | Dipole moment | OS |
|---|---|---|---|---|
| Example 1-1 | 2.79 | 2.58 | 2.93 | 0.003 |
| Example 1-2 | 2.66 | 2.42 | 3.26 | 0.0001 |
| Example 1-3 | 2.78 | 2.57 | 2.55 | 0.0108 |
| Example 1-4 | 2.69 | 2.67 | 1.11 | 0.0001 |
| Example 1-5 | 3.06 | 2.51 | 2.53 | 0.0503 |
| Example 1-6 | 2.71 | 2.42 | 3.23 | 0.0001 |
| Example 1-7 | 2.70 | 2.48 | 3.70 | 0.0008 |
| Example 1-8 | 2.69 | 2.41 | 3.61 | 0.0004 |
| Example 1-9 | 2.69 | 2.42 | 3.75 | 0.0001 |

From the HOMO/LUMO data of Table 6 and Table 7, it was identified that the compound such as 149 having high efficiency and low driving in the evaluation results on device had separated electron cloud of HOMO and LUMO when checking electron cloud and OS (oscillator strength) value depending on the substituent position of the heterocyclic compound represented by Chemical Formula 1 according to the present application. In addition, finding a trend depending on whether the OS value is small or large was difficult from the calculation results, and it was seen that interactions between the host and the dopant had an effect in addition to such electron cloud.

As seen from Table 1, it was identified that, when using a combination of the compound corresponding to Chemical Formula 1 and the compound corresponding to Chemical Formula 2, an N-type host, in the light emitting layer of the organic light emitting device, superior effects were obtained in the properties of lifetime, efficiency and driving voltage compared to when using the compound corresponding to Chemical Formula 1 alone.

When comprising the N+P Compound in the organic material layer of the organic light emitting device, an exciplex phenomenon occurs, which leads to superior results in efficiency and lifetime. The exciplex phenomenon of the N+P Compound is a phenomenon of releasing energy having sizes of a donor (p-host) HOMO level and an acceptor (n-host) LUMO level due to electron exchanges between two molecules. When a donor (p-host) having a favorable hole transfer ability and an acceptor (n-host) having a favorable electron transfer ability are used as a host of a light emitting layer, holes are injected to the p-host and electrons are injected to the n-host, and therefore, a driving voltage may be lowered, which resultantly helps with enhancement in the lifetime.

## Claims

1. A heterocyclic compound represented by the following Chemical Formula 1: 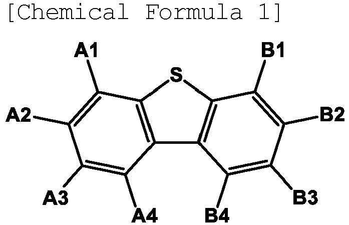 wherein, in Chemical Formula 1,
at least one of A1 to A4 is represented by the following Chemical Formula 1-1;
at least one of B1 to B4 is represented by the following Chemical Formula 1-2; and
substituents other than Chemical Formula 1-1 among A1 to A4 and substituents other than Chemical Formula 1-2 among B1 to B4 are the same as or different from each other, and each independently selected from the group consisting of hydrogen; deuterium; a halogen group; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -SiRR'R"; -P(=O)RR'; and -NRR', or two or more groups adjacent to each other bond to each other to form a substituted or unsubstituted C6 to C60 aromatic hydrocarbon ring or a substituted or unsubstituted C2 to C60 heteroring, 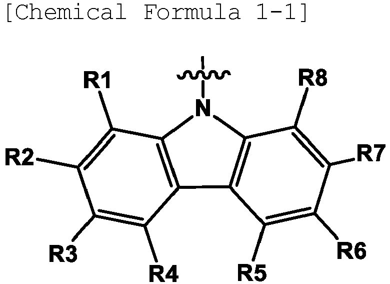 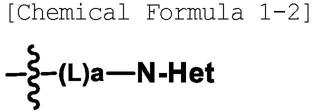
in Chemical Formula 1-1 and Chemical Formula 1-2, 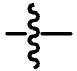 means a substituted position;
R1 to R8 are the same as or different from each other, and each independently selected from the group consisting of hydrogen; deuterium; a halogen group; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; - SiRR'R"; -P(=O)RR'; and -NRR', or two or more groups adjacent to each other bond to each other to form a substituted or unsubstituted C6 to C60 aromatic hydrocarbon ring or a substituted or unsubstituted C2 to C60 heteroring;
N-Het is a monocyclic or polycyclic C2 to C60 heterocyclic group substituted or unsubstituted and comprising one or more Ns;
L is a direct bond; a substituted or unsubstituted C6 to C60 arylene group; or a substituted or unsubstituted C2 to C60 heteroarylene group;
R, R' and R" are the same as or different from each other, and each independently hydrogen; a substituted or unsubstituted C1 to C60 alkyl group; or a substituted or unsubstituted C6 to C60 aryl group;
a is an integer of 0 to 3; and
when A3 is substituted with Chemical Formula 1-1 in Chemical Formula 1, any one of B1, B2 and B4 of Chemical Formula 1 is substituted with Chemical Formula 1-2.

2. The heterocyclic compound of Claim 1, wherein Chemical Formula 1-1 is represented by any one of the following Chemical Formulae 1-1-1 to 1-1-8: 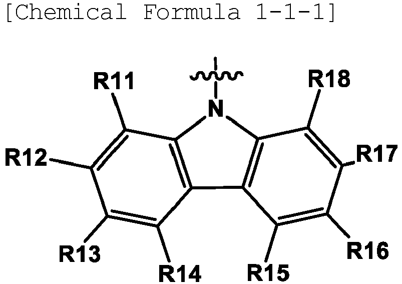 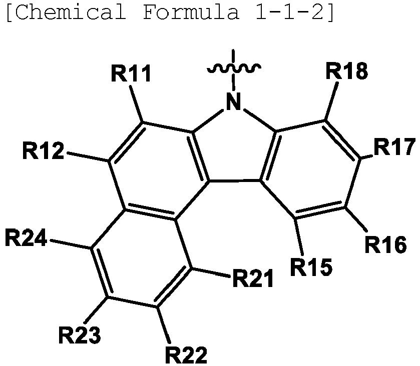 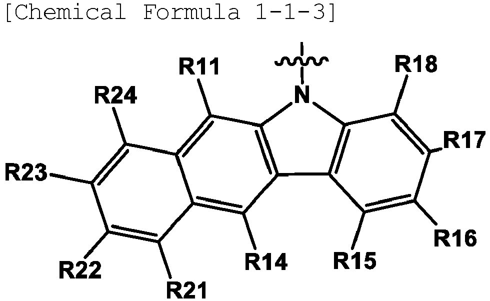 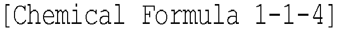 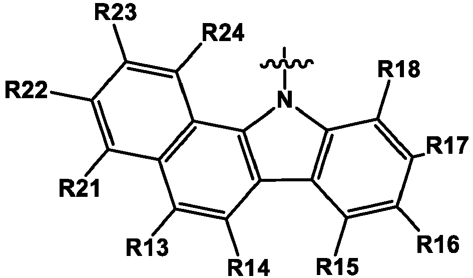 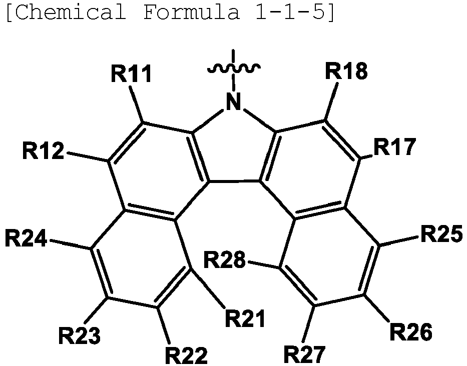 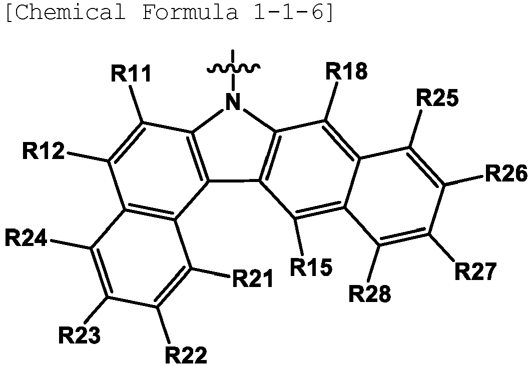 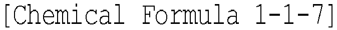 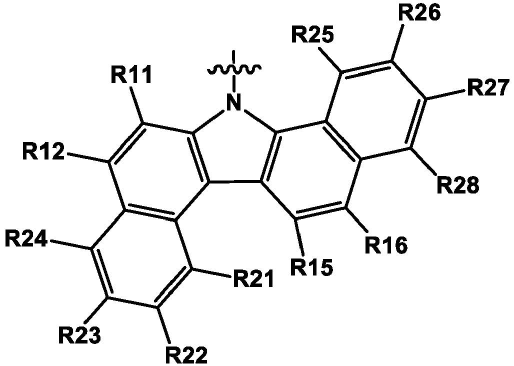 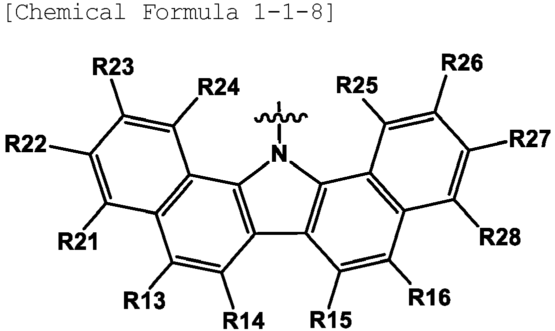 in Chemical Formulae 1-1-1 to 1-1-8,
R11 to R18 are the same as or different from each other, and each independently hydrogen; deuterium; a substituted or unsubstituted C1 to C60 alkyl group; a substituted or unsubstituted C6 to C60 aryl group; or a substituted or unsubstituted C2 to C60 heteroaryl group;
R21 to R28 are the same as or different from each other, and each independently selected from the group consisting of hydrogen; deuterium; a halogen group; a cyano group; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; - SiRR'R"; -P(=O)RR'; and -NRR', or two or more groups adjacent to each other bond to each other to form a substituted or unsubstituted C6 to C60 aromatic hydrocarbon ring or a substituted or unsubstituted C2 to C60 heteroring; and
R, R' and R" have the same definitions as in Chemical Formula 1.

3. The heterocyclic compound of Claim 1, wherein Chemical Formula 1-2 is represented by any one of the following Chemical Formulae 1-2-1 to 1-2-3: 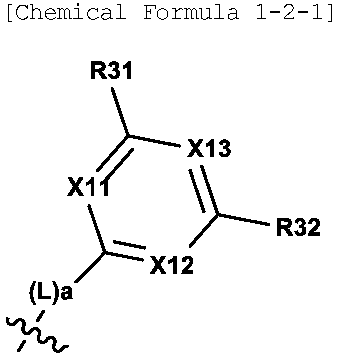 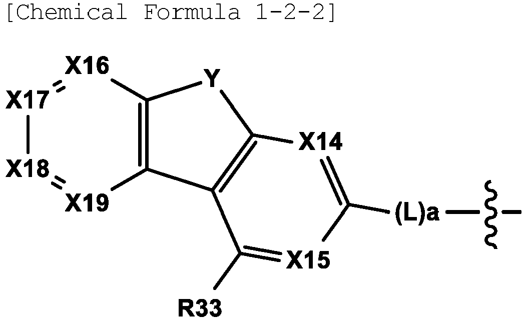 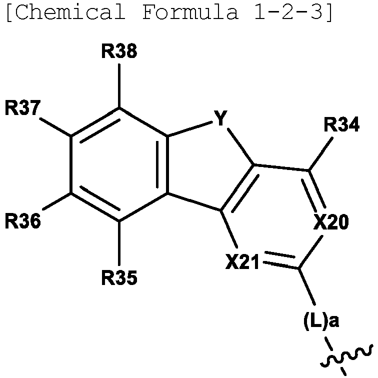 in Chemical Formulae 1-2-1 to 1-2-3,
L and a have the same definitions as in Chemical Formula 1-2; 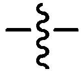 means a position linked to Chemical Formula 1;
X11 to X21 are the same as or different from each other, and each independently N; or CR40;
at least one of X11 to X13 is N, at least one of X14 to X19 is N, and at least one of X20 and X21 is N;
R40 is hydrogen; deuterium; or a substituted or unsubstituted C6 to C60 aryl group;
Y is O; or S; and
R31 to R38 are the same as or different from each other, and each independently hydrogen; deuterium; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; or a substituted or unsubstituted C2 to C60 heteroaryl group, or two or more groups adjacent to each other bond to each other to form a substituted or unsubstituted C6 to C60 aliphatic or aromatic hydrocarbon ring or a substituted or unsubstituted C2 to C60 aliphatic or aromatic heteroring.

4. The heterocyclic compound of Claim 1, wherein the substituents other than Chemical Formula 1-1 among A1 to A4 and the substituents other than Chemical Formula 1-2 among B1 to B4 are hydrogen.

5. The heterocyclic compound of Claim 1, wherein Chemical Formula 1 is represented by any one of the following compounds:

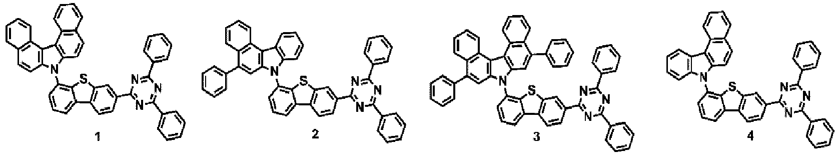

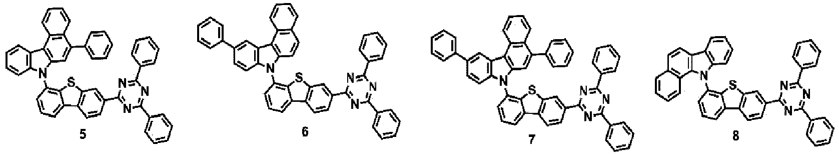

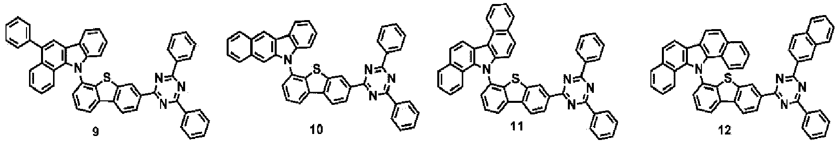

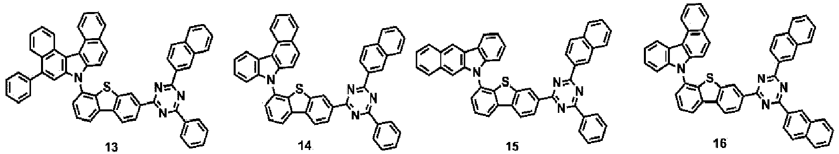

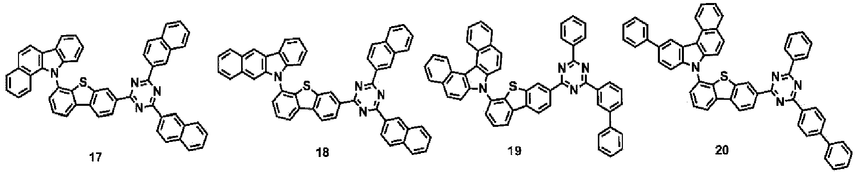

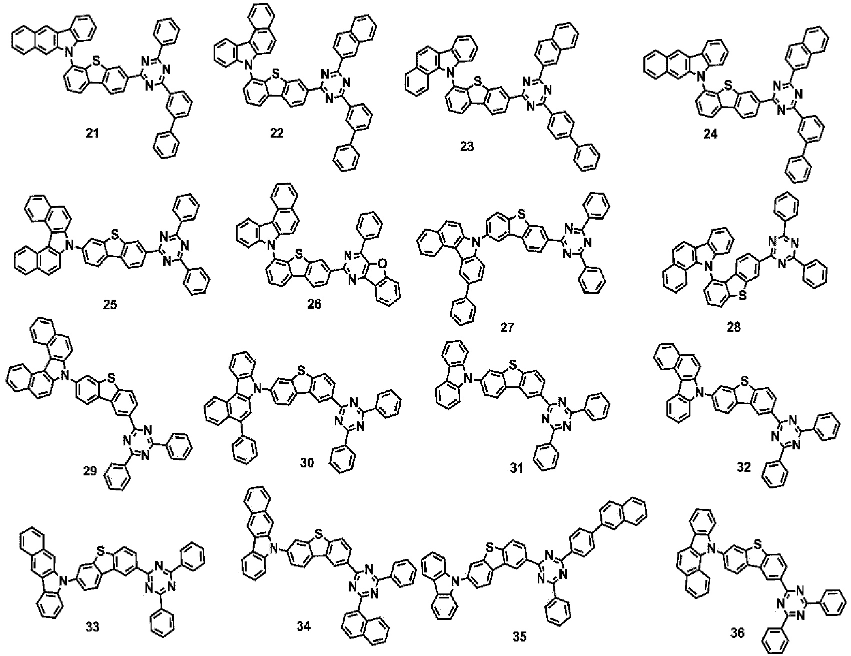

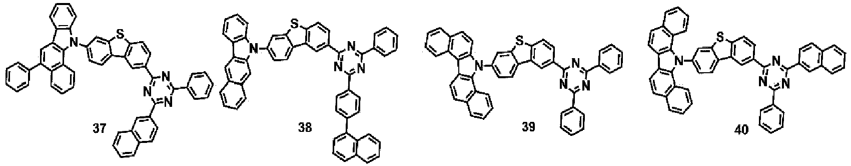

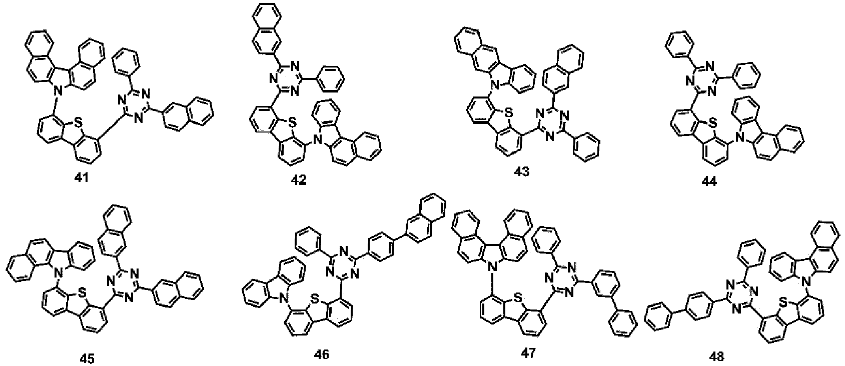

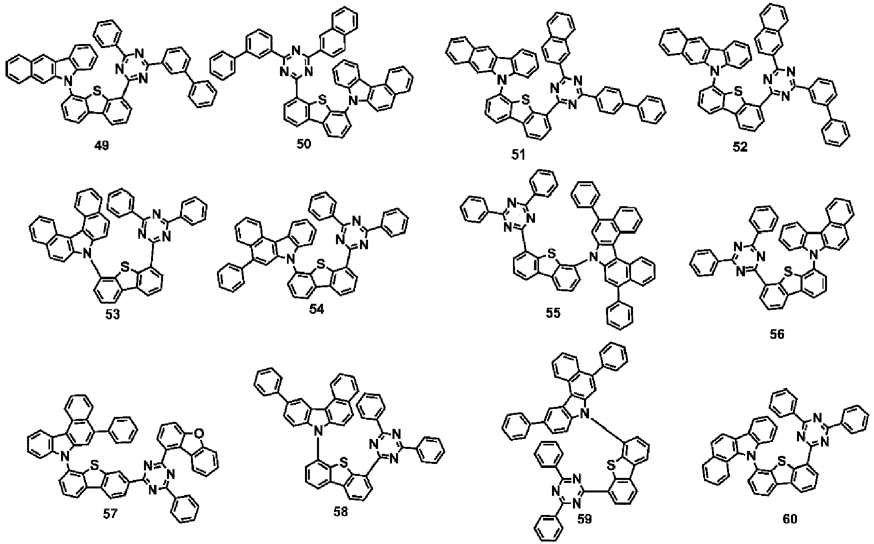

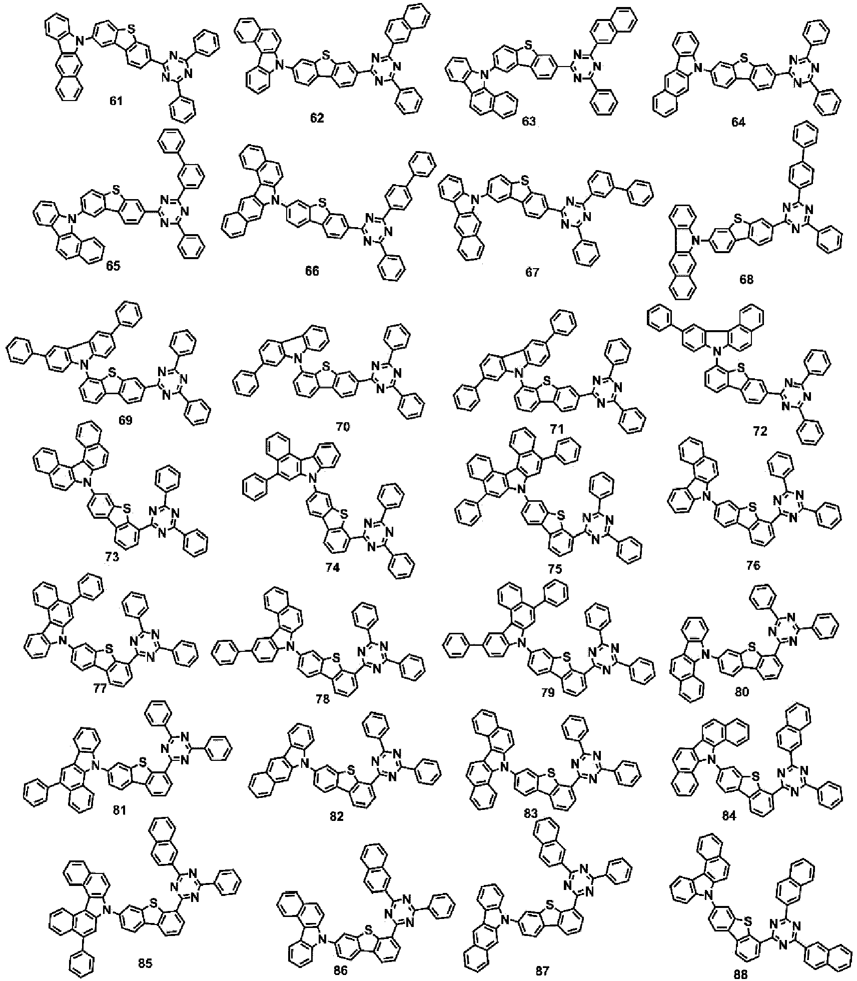

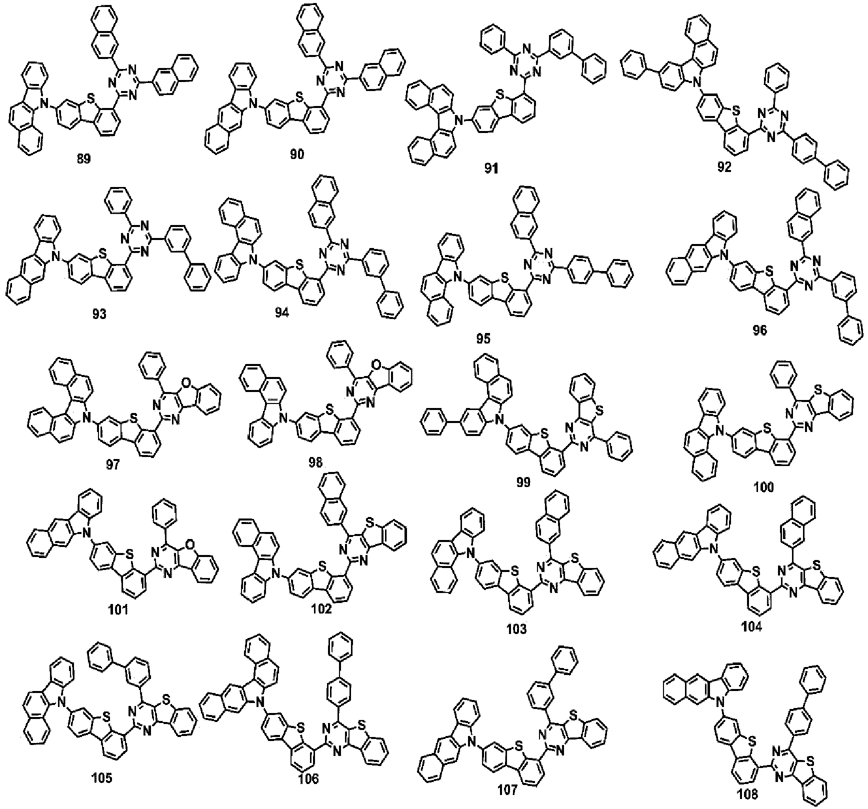

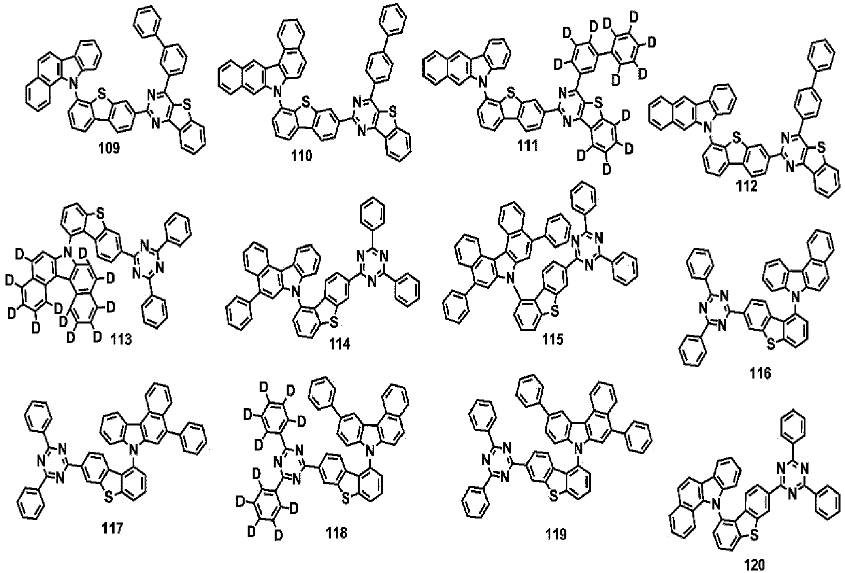

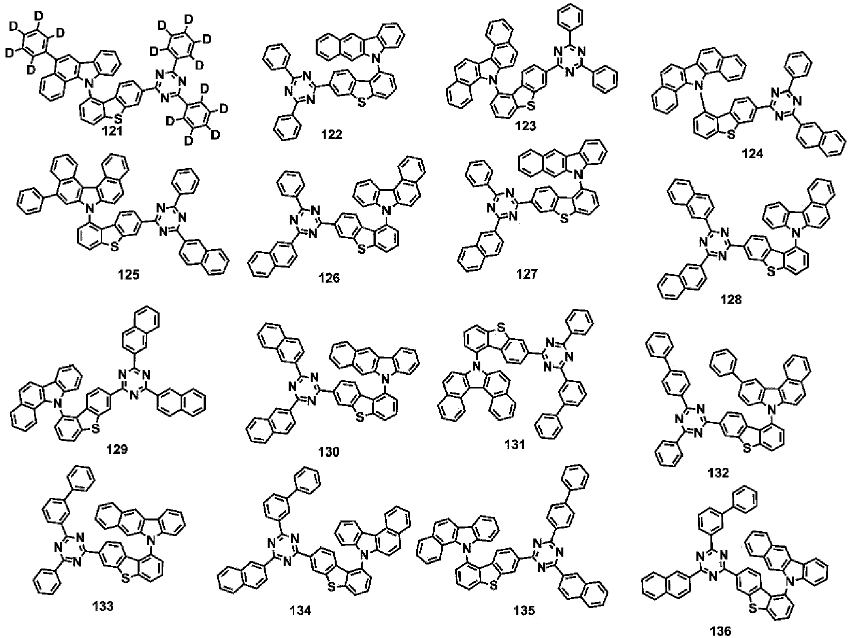

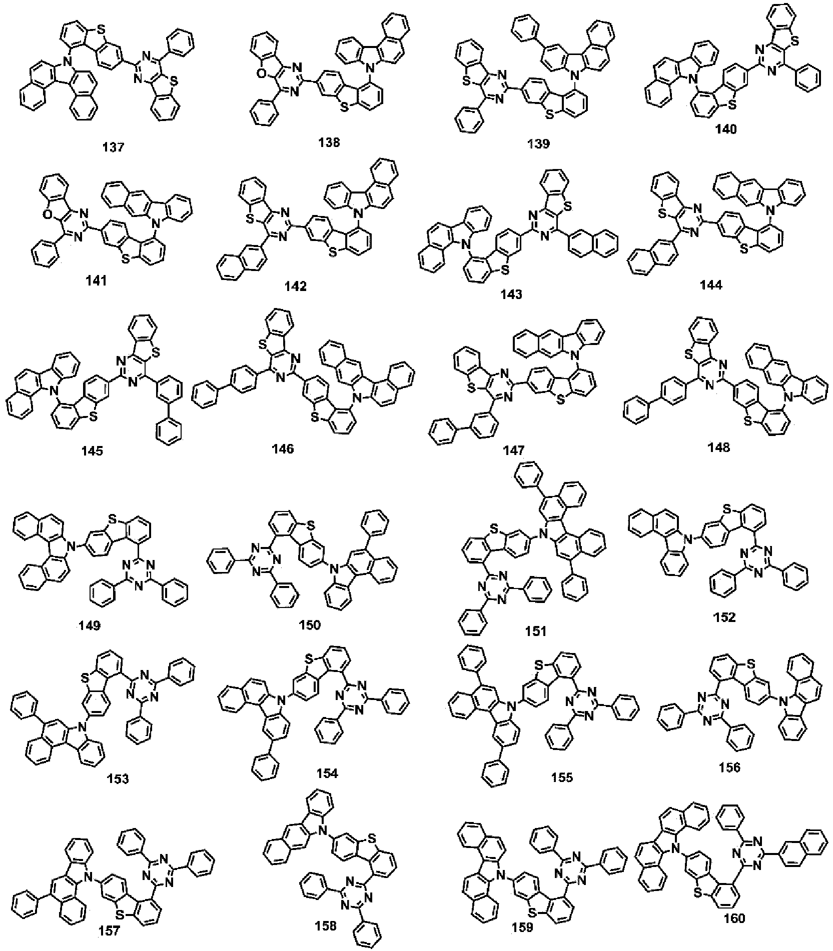

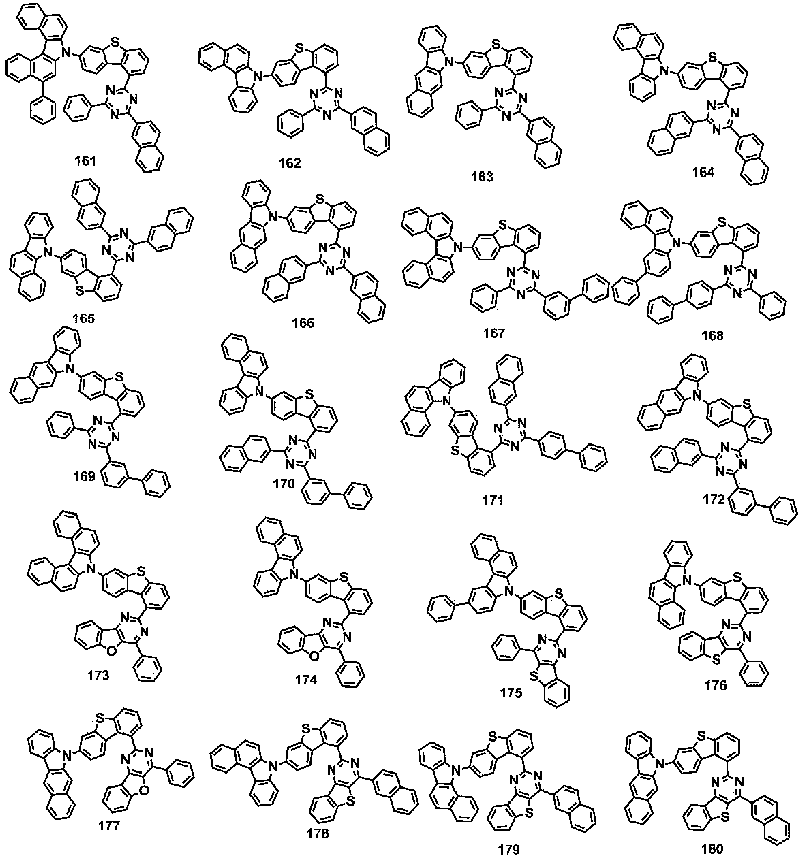

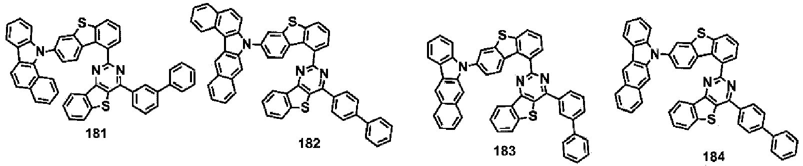

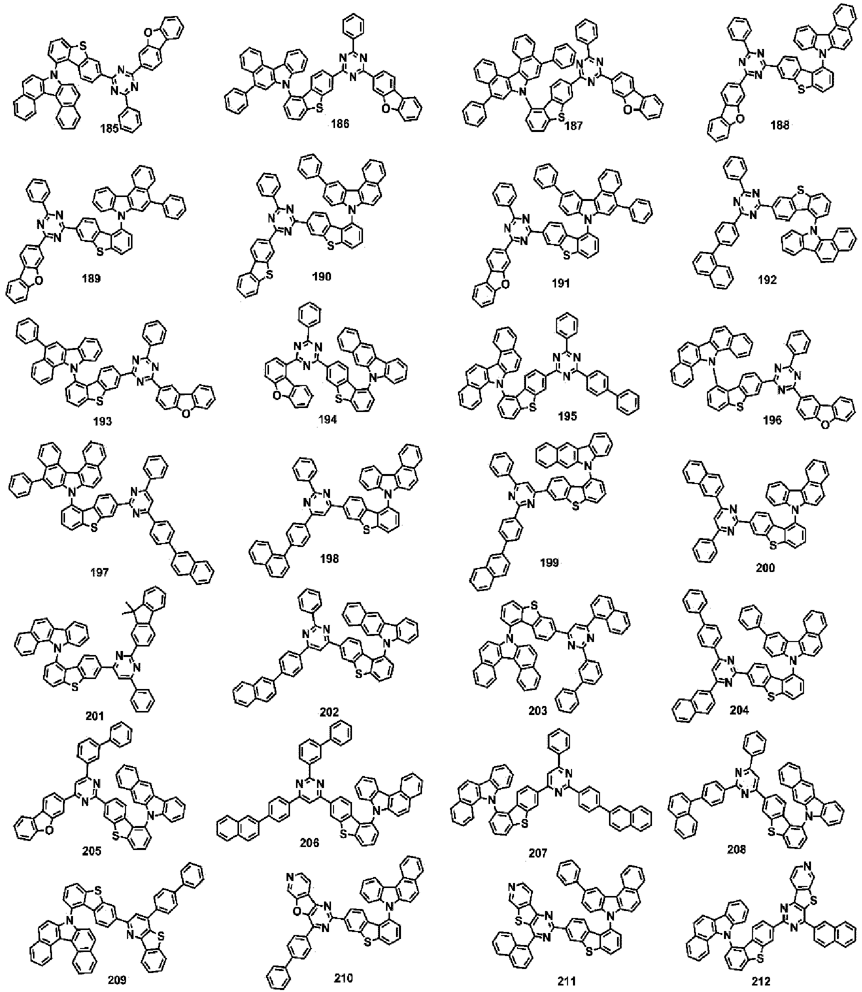

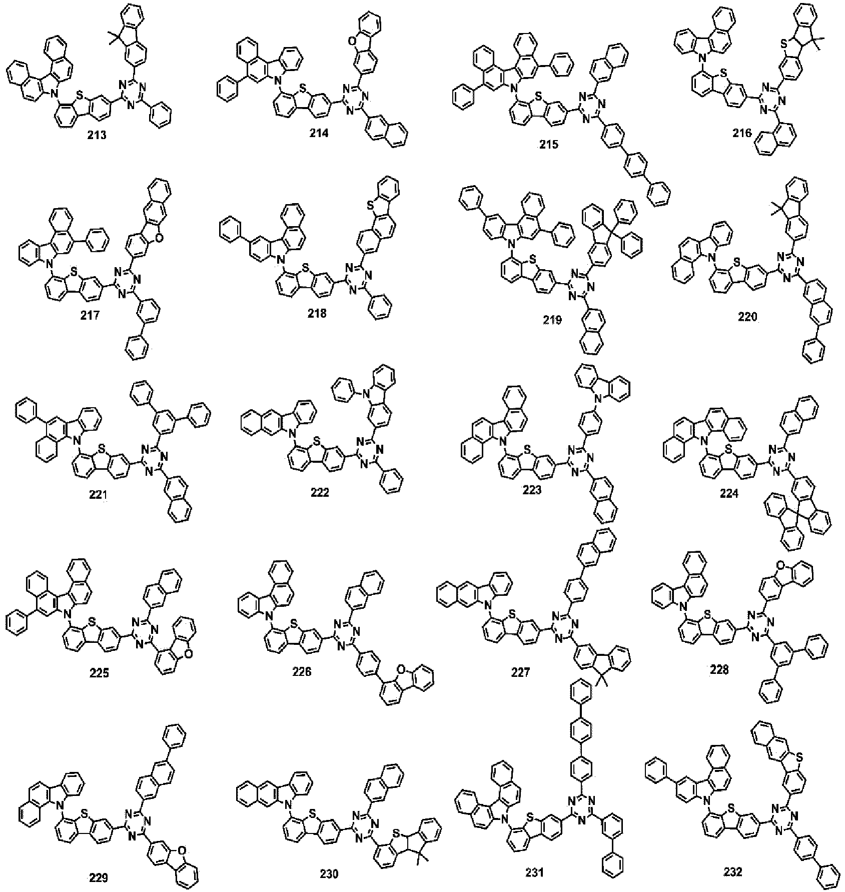

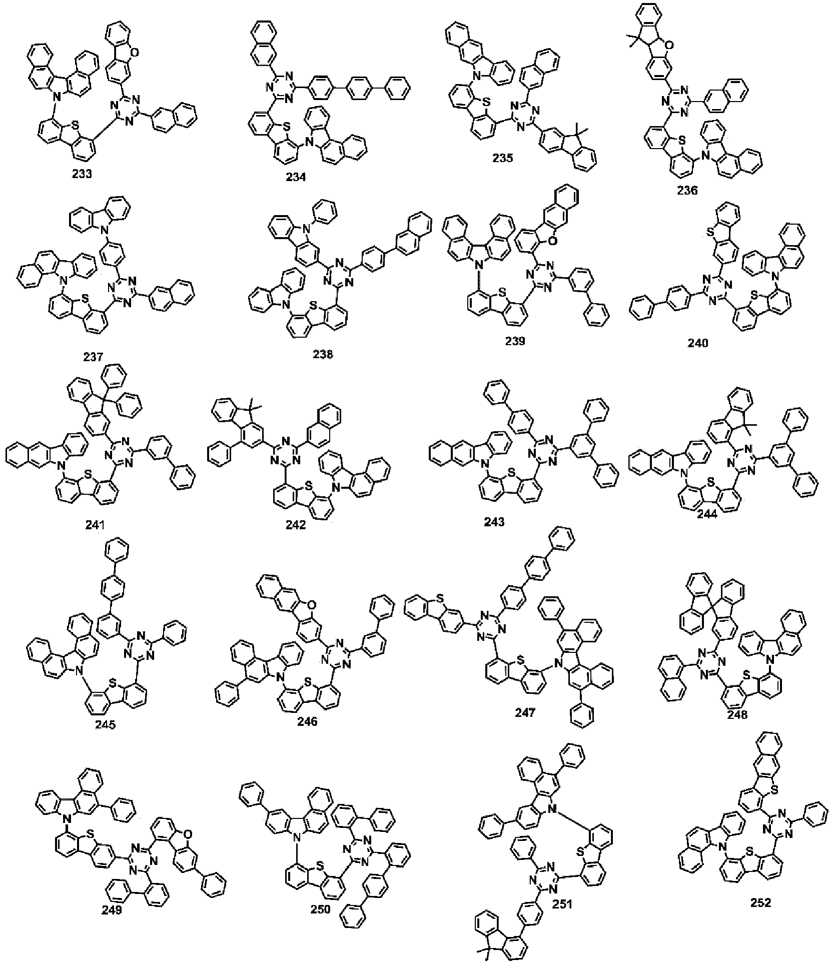

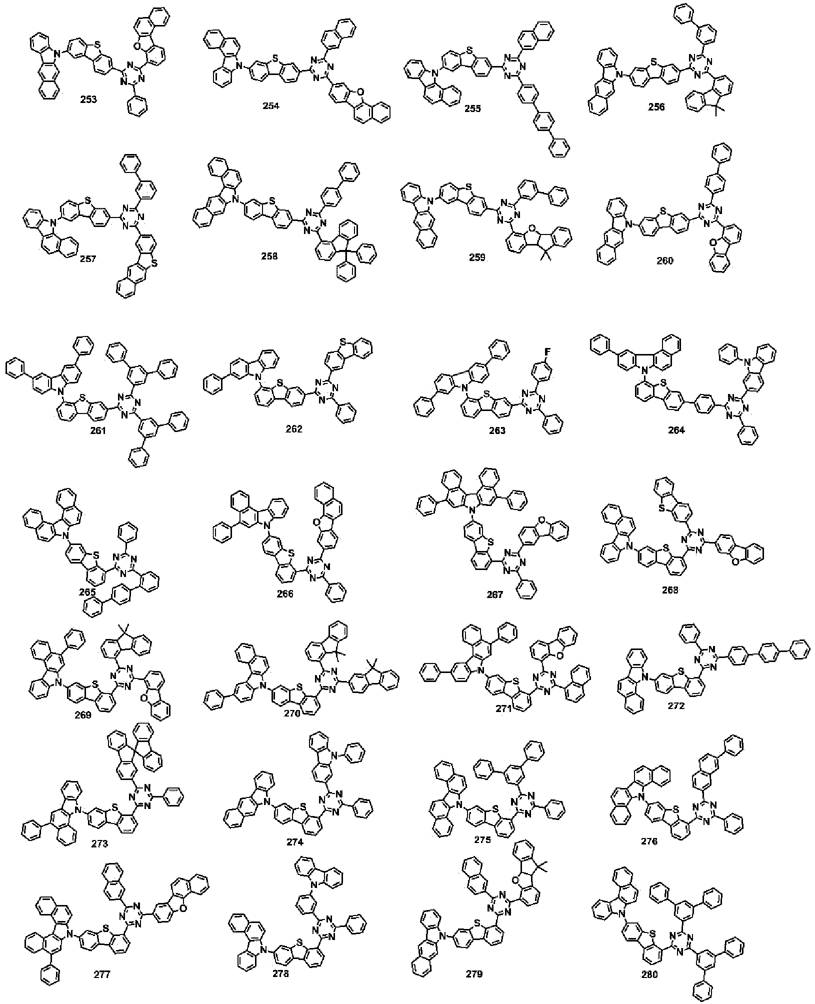

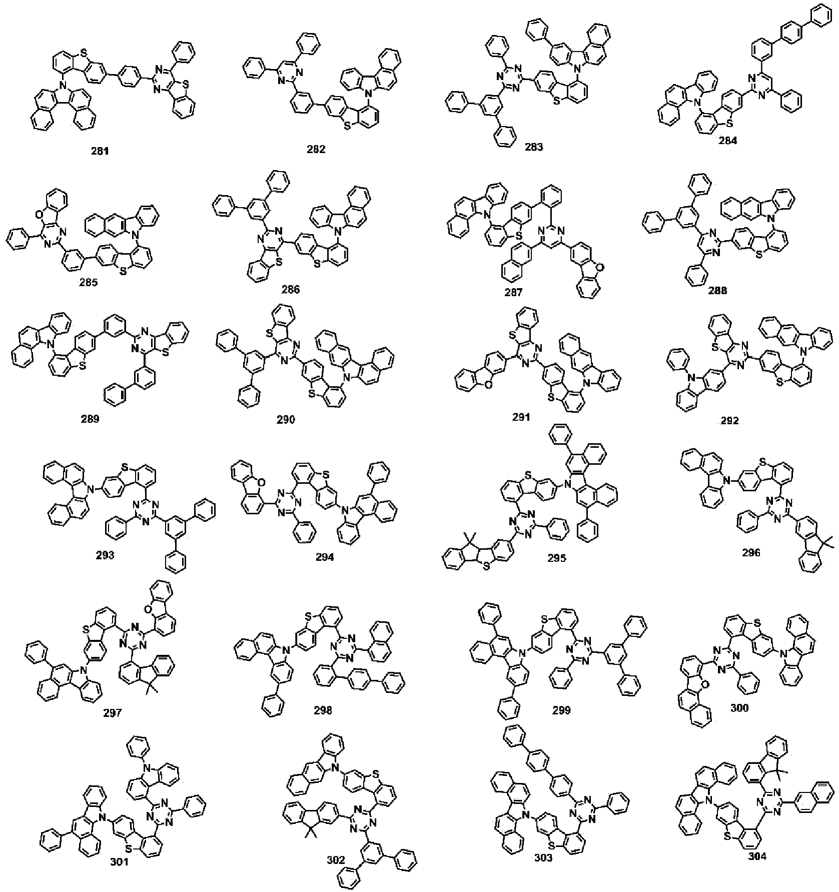

6. An organic light emitting device comprising:
a first electrode;
a second electrode provided opposite to the first electrode; and
one or more organic material layers provided between the first electrode and the second electrode,
wherein one or more layers of the organic material layers comprise the heterocyclic compound of any one of Claims 1 to 5.

7. The organic light emitting device of Claim 6, wherein the organic material layer comprising the heterocyclic compound further comprises a heterocyclic compound represented by the following Chemical Formula 2: 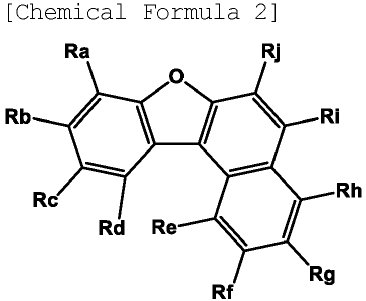 in Chemical Formula 2,
Ra to Rj are the same as or different from each other, and each independently selected from the group consisting of hydrogen; deuterium; a halogen group; -CN; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -SiR101R102R103; - P(=O)R101R102; and -NR101R102, or two or more groups adjacent to each other bond to each other to form a substituted or unsubstituted C6 to C60 aromatic hydrocarbon ring or a substituted or unsubstituted C2 to C60 heteroring;
R101, R102 and R103 are the same as or different from each other, and each independently hydrogen; deuterium; -CN; a substituted or unsubstituted C1 to C60 alkyl group; a substituted or unsubstituted C6 to C60 aryl group; or a substituted or unsubstituted C2 to C60 heteroaryl group; and
at least one of Ra to Rj is a substituted or unsubstituted C2 to C60 heteroaryl group.

8. The organic light emitting device of Claim 7, wherein Chemical Formula 2 is represented by the following Chemical Formula 2-1: 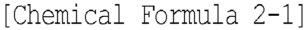 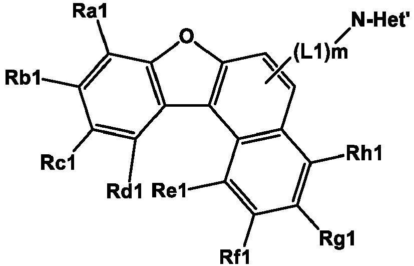 in Chemical Formula 2-1,
Ra1 to Rh1 are each independently selected from the group consisting of hydrogen; a substituted or unsubstituted C1 to C60 alkyl group; a substituted or unsubstituted C6 to C60 aryl group; and a substituted or unsubstituted C2 to C60 heteroaryl group;
L1 is a direct bond; a substituted or unsubstituted arylene group; or a substituted or unsubstituted heteroarylene group;
N-Het' is a monocyclic or polycyclic C2 to C60 heterocyclic group substituted or unsubstituted and comprising one or more Ns; and
m is an integer of 0 to 4.

9. The organic light emitting device of Claim 6, wherein the organic material layer comprises a light emitting layer, and the light emitting layer comprises the heterocyclic compound of Chemical Formula 1.

10. The organic light emitting device of Claim 6, wherein the organic material layer comprises a light emitting layer, the light emitting layer comprises a host material, and the host material comprises the heterocyclic compound of Chemical Formula 1.

11. The organic light emitting device of Claim 6, further comprising one, two or more layers selected from the group consisting of a light emitting layer, a hole injection layer, a hole transfer layer, an electron injection layer, an electron transfer layer, an electron blocking layer and a hole blocking layer.

12. A composition for an organic material layer of an organic light emitting device, the composition comprising:
the heterocyclic compound of any one of Claims 1 to 5; and
a heterocyclic compound represented by the following Chemical Formula 2: 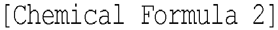 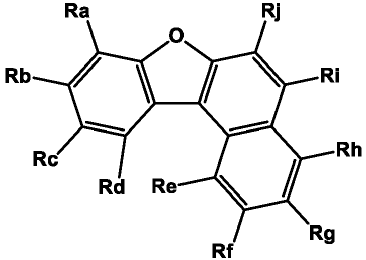 wherein, in Chemical Formula 2,
Ra to Rj are the same as or different from each other, and each independently selected from the group consisting of hydrogen; deuterium; a halogen group; -CN; a substituted or unsubstituted C1 to C60 alkyl group; a substituted or unsubstituted C2 to C60 alkenyl group; a substituted or unsubstituted C2 to C60 alkynyl group; a substituted or unsubstituted C1 to C60 alkoxy group; a substituted or unsubstituted C3 to C60 cycloalkyl group; a substituted or unsubstituted C2 to C60 heterocycloalkyl group; a substituted or unsubstituted C6 to C60 aryl group; a substituted or unsubstituted C2 to C60 heteroaryl group; -SiR101R102R103; - P(=O)R101R102; and -NR101R102, or two or more groups adjacent to each other bond to each other to form a substituted or unsubstituted C6 to C60 aromatic hydrocarbon ring or a substituted or unsubstituted C2 to C60 heteroring;
R101, R102 and R103 are the same as or different from each other, and each independently hydrogen; deuterium; -CN; a substituted or unsubstituted C1 to C60 alkyl group; a substituted or unsubstituted C6 to C60 aryl group; or a substituted or unsubstituted C2 to C60 heteroaryl group; and
at least one of Ra to Rj is a substituted or unsubstituted C2 to C60 heteroaryl group.

13. The composition for an organic material layer of an organic light emitting device of Claim 12, wherein, in the composition, the heterocyclic compound represented by Chemical Formula 1:the heterocyclic compound represented by Chemical Formula 2 have a weight ratio of 1:10 to 10:1.

14. A method for manufacturing an organic light emitting device, the method comprising:
preparing a substrate;
forming a first electrode on the substrate;
forming one or more organic material layers on the first electrode; and
forming a second electrode on the organic material layer,
wherein the forming of organic material layers comprises forming one or more organic material layers using the composition for an organic material layer of Claim 12.

15. The method for manufacturing an organic light emitting device of Claim 14, wherein the forming of organic material layers is forming using a thermal vacuum deposition method after pre-mixing the heterocyclic compound represented by Chemical Formula 1 and the heterocyclic compound represented by Chemical Formula 2.
